(19) Europäisches Patentamt
European Patent Office
Office européen des brevets

(11) **EP 2 754 405 A1**

(12) **EUROPEAN PATENT APPLICATION**

(43) Date of publication:
16.07.2014 Bulletin 2014/29

(51) Int Cl.:
*A61B 19/00* ^(2006.01)

(21) Application number: 14151130.3

(22) Date of filing: 14.01.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 15.01.2013 US 201361752474 P

(71) Applicant: M.S.T. Medical Surgery Technologies Ltd.
20692 Yoqneam (IL)

(72) Inventors:
• **Sholev, Mordehai**
**37830 Moshav Amikam (IL)**
• **Atarot, Gal**
**44646 Kfar Saba (IL)**
• **Karvat, Shlomi**
**19125 Ramat Zvi (IL)**

(74) Representative: **Lecomte & Partners**
**P.O. Box 1623**
**1016 Luxembourg (LU)**

(54) **A device for maneuvering endoscope**

(57) The present invention provides a system and method for maneuvering an endoscope. The system comprises at least one first pivoting support adapted to be pivotally attached to an endoscope. Further, at least one second pivoting support is provided, which is in communication with said at least one first pivoting support. A controller is adapted to provide a constant dynamic equilibrium between the endoscope and at least one of the first and second pivoting supports.

EP 2 754 405 A1

**Description**

**FIELD OF THE INVENTION**

The present invention generally relates to means and methods for simply maneuvering an endoscope by an endoscope user. Moreover, this present invention discloses a compact configuration of devices used for different actions upon the endoscope.

**BACKGROUND OF THE INVENTION**

In laparoscopic surgery, the surgeon performs the operation through small holes using long instruments and observing the internal anatomy with an endoscope camera. The endoscope is conventionally held by a camera assistant since the surgeon must perform the operation using both hands. The surgeon's performance is largely dependent on the camera's position relative to the instruments and on a stable image shown by the monitor; also the picture shown must be in the right orientation. The main problem is the difficulty for the assistant in keeping the endoscope in the right spatial position, holding the endoscope steadily, and keeping the scene in the right orientation. To overcome these problems, several new technologies have been developed, using robots to hold the endoscope while the surgeon performs the procedure, e.g., Lapman, Endoassist, etc. But these technologies are expensive, difficult to install, uncomfortable to use, limit the dexterity of the surgeon and have physical dimension much bigger that all the operating tools. Relative to the required action, they also move in big increments with several arms moving. Another robot, LER, (which was developed by the TIMC-GMCAO Laboratory) is described in US. Patent application No. 200/6100501. It consists of a compact camera-holder robot that rests directly on the patient's abdomen and an electronic box containing the electricity supply and robot controllers. LER has relatively small dimensions but has a 110 mm diameter base ring that must be attached, or be very close to, the patient's skin. This ring occupies space over the patient's body, affecting the surgeon's activities: limiting the surgeon's choice of where to place other trocars, changing the surgeon's usual way of making the procedure, sometimes forcing the setup process to be as long as 40 minutes. Also the LER has only 3 degrees of freedom and has no ability to control the orientation of the picture shown to surgeon (the LER cannot rotate the endoscope around its longitudinal axis). Laparoscopic surgery is becoming increasingly popular with patients because the scars are smaller and their period of recovery is shorter. Laparoscopic surgery requires special training for the surgeon or gynecologist and the theatre nursing staff. The equipment is often expensive and is not available in all hospitals. During laparoscopic surgery, it is often required to shift the spatial placement of the endoscope in order to present the surgeon with an optimal view. Conventional laparoscopic surgery makes use of either human assistants that manually shift the instrumentation or alternatively robotic automated assistants (such as JP patent No. 06063003).
However, even the improved technologies still limit the dexterity of the surgeon and fail to provide four degrees of freedom. Another disadvantage of those technologies is the lack of ability to control the spatial position of an endoscope tube to any orientation during the laparoscopic surgery, such that the surgeon reaches any desired area within the working envelope in the body being operated on.
Therefore, there is still a long felt need for a camera holder that would allow holding and controlling the endoscope steady without limiting the dexterity of the surgeon and that will provide four degrees of freedom. Furthermore, there is still a long felt need for a camera holder that will provide the ability to control the spatial position of an endoscope tube to any orientation during the laparoscopic surgery, such that the surgeon reaches any desired area within the working envelope in operated body, without putting pressure on the penetration point where the endoscope enters the body.

**SUMMARY OF THE INVENTION**

An object of the invention is to disclose a system for maneuvering an endoscope, comprising:

a. at least one first pivoting support adapted to be pivotally attached to the endoscope; the pivoting support adapted to enable the endoscope to pivot around at least one first axis of rotation;
b. at least one second pivoting support in communication with the at least one first pivoting support, the second pivoting support adapted to rotate around at least one axis being substantially orthogonal to the first axis of rotation independently of the first pivoting support; thereby enabling the endoscope to rotate around an insertion point into a body of a subject in at least two orthogonal axes;
c. at least one controller attached to either the first pivoting support or the second pivoting support, wherein the controller is adapted to provide a constant dynamic equilibrium between the endoscope and at least one of the first pivoting support or the second pivoting support.

It is another object of the invention is to disclose the system as defined above, wherein at least one of the pivoting supports is adapted to moderate the pivoting of the endoscope around either the first axis of rotation or the second axis of rotation.

It is another object of the invention is to disclose the system as defined above, wherein the system further comprises at least one connecting means adapted to connect the endoscope to at least one of the pivoting supports.

It is another object of the invention is to disclose the system as defined above, wherein at least one of the pivoting supports is adapted to constantly apply a counter torque to oppose the torque induced by the endoscope and the connecting means.

It is another object of the invention is to disclose the system as defined above, wherein the counter torque varies as the torque varies such that there is provided a constant dynamic equilibrium between the endoscope and at least one of the first pivoting support and the second pivoting support.

It is another object of the invention is to disclose the system as defined above, wherein the first pivoting support is adapted to enable the endoscope to pivot around one first axis of rotation and the second pivoting support is adapted to enable the endoscope to pivot around a second and third axis of rotation, each of the first axis of rotation, the second axis of rotation and the third axis of rotation being substantially perpendicular to the other two axes of rotation.

It is another object of the invention is to disclose the system as defined above, wherein the controller further comprises a damping mechanism adapted to prevent oscillation of the system.

It is another object of the invention is to disclose the system as defined above, wherein the controller comprises at least one of an active mechanism and a passive mechanism to provide the dynamic equilibrium.

It is another object of the invention is to disclose the system as defined above, wherein the controller comprises at least one selected from a group consisting of a motor and a spring.

It is another object of the invention is to disclose the system as defined above, wherein the torque applied by the controller increases as the angle between the vertical and the endoscope increases.

It is another object of the invention is to disclose the system as defined above, wherein activation of the control is at least one of a group consisting of: the control is activated at all times, the control is activated when the endoscope's angle is greater than a predetermined value, and the control is activated when the torque on the endoscope is greater than a predetermined value.

It is another object of the invention is to disclose the system as defined above, wherein the system additionally comprises an automatic assistant in communication with the endoscope.

It is another object of the invention is to disclose the system as defined above, wherein the automatic assistant is adapted to maneuver the endoscope.

It is another object of the invention is to disclose the system as defined above, wherein the system additionally comprises an alignment mechanism.

It is another object of the invention is to disclose the system as defined above, wherein the alignment mechanism is adapted to instruct the automatic assistante to align the endoscope.

It is another object of the invention to disclose the system as defined above, further comprising:

    a. a first mechanism, comprising:

        i. at least one first transmission means **101;** first transmission means **101** defines a first plane; first transmission means **101** is characterized by a first axis of rotation; the first axis of rotation is substantially orthogonal to the first plane;
        ii. at least one second transmission means **102;** second transmission means **102** defines a second plane; the second transmission means is characterized by a second axis of rotation **141;** second axis of rotation **141** is substantially orthogonal to the second plane; second transmission means **102** is rotatably connected to first transmission means **101;** where the first plane is substantially orthogonal to second plane; and
        iii. at least one first means **106** adapted to rotate first transmission means **101** around the first axis of rotation;

    where first transmission means **101** transmits rotation to second transmission means **102;** and,
    b. a second mechanism, comprising:

        i. at least one third transmission means +**103;** third transmission means 103 defines a third plane; third transmission means **103** is characterized by a third axis of rotation; the third axis of rotation is substantially orthogonal to the third plane;
        ii. at least one fourth transmission means **104;** fourth transmission means **104** defines a fourth plane; fourth transmission means **104** defines a fourth axis of rotation; the fourth axis of rotation is substantially orthogonal to fourth plane; fourth transmission means **104** is rotatably connected to third transmission means **103;** where the fourth plane is substantially orthogonal to the third plane;
        iii. at least one fifth transmission means **105; fifth** transmission means **105** defines a fifth plane; fifth transmission means **105** defines a fifth axis of rotation **142**; fifth axis of rotation **142** is substantially orthogonal to the fifth plane; fifth transmission means **105** is rotatably connected to fourth transmission means **104**; where the fifth plane is substantially orthogonal to the fourth plane;

iv. at least one second means **107** adapted to rotate third transmission means **103** around the third axis of rotation;

where third transmission means **103** transmits rotation to fourth transmission means **104**; where fourth transmission means **104** transmits rotation to fifth transmission means **105**.

wherein the first mechanism and the second mechanism are adapted to rotate the endoscope around at least one second axis of rotation **141** being substantially orthogonal to the second plane; and around at least one fifth axis of rotation **142** being substantially orthogonal to the fifth plane, such that second axis of rotation **141** and fifth axis of rotation **142** are positioned at an angle A relative to each other.

It is another object of the invention to disclose the system as defined above, wherein angle A between second axis of rotation **141** and fifth axis of rotation **142** is in the range of about 0 degrees to about 180 degrees.

It is another object of the invention to disclose the system as defined above, further comprising at least one zoom mechanism **115,** adapted to maneuver the endoscope along its main longitudinal axis.

It is another object of the invention to disclose the system as defined above, wherein either one of the first or second pivoting supports **(113** and **114)** is a gimbal.

It is another object of the invention to disclose the system as defined above, wherein first transmission means **101,** second transmission means **102,** third transmission means **103,** fourth transmission means **104,** and fifth transmission means **105** are selected from a group consisting of gearwheels, wheels, crown gears, bevel gears, spur gears, belts, and any combination thereof.

It is another object of the invention to disclose the system as defined above, wherein the system comprises attaching means adapted to reversibly couple the system to a hospital bed, the attaching means selected from a group consisting of mechanical means, magnetic means and any combination thereof.

It is another object of the invention to disclose the system as defined above, wherein the mechanical means is selected from a group consisting of a clip, a fastening element, tape, adhesive tape, a snap fastener, a button and any combination thereof.

It is another object of the invention to disclose the system as defined above, wherein the magnetic means comprises a magnetic device, the magnetic device comprising at least one magnet and at least one selected from a group consisting of: a ferromagnet and a paramagnet; where the magnetic is attached to at least one member of a group consisting of: a hospital bed, the system, and any combination thereof, and the member of the group consisting of a ferromagnet and a paramagnet is attached to at least one member of a group consisting of: a hospital bed, the system, and any combination thereof.

It is another object of the invention to disclose the system as defined above, wherein a rotation in the second plane defines an angle $\theta$.

It is another object of the invention to disclose the system as defined above, wherein the angle $\theta$ varies between about 0 and about 360 degrees, preferably between about 0 and about 160 degrees, when the system is in a automatic configuration or in a manual configuration.

It is another object of the invention to disclose the system as defined above, wherein a rotation in the fifth plane defines an angle $\psi$.

It is another object of the invention to disclose the system as defined above, wherein the angle $\psi$ varies between about 0 and about 360 degrees, preferably between about 0 and about 140 degrees, when system is in its automatic configuration or in its manual configuration.

It is another object of the invention to disclose the system as defined above, wherein the system additionally comprises a quick release handle adapted to disassemble the endoscope from the system when the system is in either its automatic configuration or its manual configuration.

It is another object of the invention to disclose the system as defined above, wherein the first mechanism additionally comprises locking means adapted to maintain in a predetermined orientation upon power failure at least one selected from a group consisting of: the first transmission means, the second transmission means and any combination thereof; and to prevent any rotational movement of the same upon power failure.

It is another object of the invention to disclose the system as defined above, wherein the second mechanism additionally comprises locking means adapted to maintain in a predetermined orientation upon power failure at least one selected from a group consisting of: the third transmission means, the fourth transmission means, the fifth transmission means, and any combination thereof; and to prevent any rotational movement of the same upon power failure.

It is another object of the invention to disclose the system as defined above, additionally comprising at least one manual override system (MOS), adapted upon activation of the same to switch reversibly between a manual configuration, in which the endoscope is moved manually by the operator and an automatic configuration, in which the endoscope is moved automatically by the system.

It is another object of the invention to disclose the system as defined above, additionally comprising at least one joystick, in communication with the endoscope.

It is another object of the invention to disclose the system as defined above, additionally comprising activation means adapted to activate at least one of a group consisting of the system, the joystick and any combination thereof.

It is another object of the invention to disclose the system as defined above, wherein the activation means is adapted to be worn by the MOS operator.

It is another object of the invention to disclose the system as defined above, adapted to be worn by the joystick user.

It is another object of the invention to disclose the system as defined above, wherein the activation means is selected from a group consisting of a pressable button, a rotatable knob, a knob, and any combination thereof.

It is another object of the invention to disclose the system as defined above, wherein the MOS enables rotation in the angles $\psi$ and $\theta$.

It is another object of the invention to disclose the system as defined above, wherein, when the joystick is moved in direction $\alpha$, the endoscope moves in angular direction $\theta$ and when the joystick is moved in direction $\beta$, the endoscope moves in angular direction $\psi$.

It is another object of the invention to disclose the system as defined above, wherein movement of the joystick in a direction selected from a group consisting of $\alpha$, $\beta$ and any combination thereof is proportional to movement of the endoscope in a direction selected from a group consisting of $\psi$, $\theta$ and any combination thereof.

It is another object of the invention to disclose the system as defined above, wherein the MOS additionally comprises means for controlling the endoscope's motion, adapted to moderate angular velocity in the $\theta$ and $\psi$ directions.

It is another object of the invention to disclose the system as defined above, wherein the MOS additionally comprises *n* sensors, where n is an integer greater than or equal to one.

It is another object of the invention to disclose the system as defined above, wherein the sensors are selected from of a group consisting of motion sensors, heat sensors, electric sensors, sound sensors, pressure sensors, optical sensors and any combination thereof.

It is another object of the invention to disclose the system as defined above, wherein the *n* sensors are activated in at least one selected from a group consisting of: in case of power failure and when the system is connected to power.

It is another object of the invention to disclose the system as defined above, wherein the motion sensors detect motion of the joystick.

It is another object of the invention to disclose the system as defined above, wherein the motion detection of the joystick is used to deactivate the motion of the endoscope if the motion's speed is above a predetermined threshold.

It is another object of the invention to disclose the system as defined above, wherein the joystick is characterized by an external surface.

It is another object of the invention to disclose the system as defined above, wherein the motion sensors detect motion upon the external surface.

It is another object of the invention to disclose the system as defined above, wherein the motion upon the external surface is used to operate the endoscope according to the motion upon the external surface.

It is another object of the invention to disclose the system as defined above, wherein the motion upon the external surface deactivates of the motion of the endoscope if the motion's speed is above a predetermined threshold.

It is another object of the invention to disclose the system as defined above, wherein the heat sensors are adapted to sense temperature in the range of about 35 to about 42 degrees.

It is another object of the invention to disclose the system as defined above, wherein the heat sensors are adapted to provide a thermal image, where the heat sensors are coupled to a processing unit adapted to provide the endoscope user with the thermal image.

It is another object of the invention to disclose the system as defined above, wherein the heat sensors enable activation of the MOS when the heat sensors sense a temperature is in the range of about 35 to about 42 degrees.

It is another object of the invention to disclose the system as defined above, wherein the electric sensors are adapted to sense at least one of a group consisting of: power failure, connection to power, and electrical conductivity of a human body.

It is another object of the invention to disclose the system as defined above, wherein the sound sensors are adapted to sense predetermined sound patterns..

It is another object of the invention to disclose the system as defined above, wherein the sound sensors are used to operate the endoscope according to the predetermined sound patterns.

It is another object of the invention to disclose the system as defined above, wherein the optical sensors are adapted to sense visual changes according to predetermined visual patterns.

It is another object of the invention to disclose the system as defined above wherein the optical sensors are used to operate the endoscope according to the predetermined visual patterns.

It is another object of the invention to disclose the system as defined above, wherein the pressure sensors are adapted to sense pressure applied to the MOS.

It is another object of the invention to disclose the system as defined above, wherein the MOS is activated upon at least one condition selected from a group consisting of: analysis of the thermal image by the processing unit and detection of human hand, the electric sensors sense human body conductivity, the sound sensors sense predetermined sound patterns, and according to predetermined visual patterns detected by the optical sensors.

It is another object of the invention to disclose the system as defined above, wherein the MOS is adapted to change its activation state in a manner selected from at least one of a group consisting of: when the pressure sensed by the pressure sensors is above a predetermined threshold the MOS is activated, and when the pressure sensed by the pressure sensors is below a predetermined threshold, the MOS is de-activated.

It is an object of the invention to disclose a method for maneuvering an endoscope, the method comprising steps of:

  a. providing a system comprising:

    i. at least one first pivoting support adapted to be pivotally attached to the endoscope; the pivoting support adapted to enable the endoscope to pivot around at least one first axis of rotation;
    ii. at least one second pivoting support in communication with the at least one first pivoting support, the second pivoting support adapted to rotate around at least one axis being substantially orthogonal to the first axis of rotation independently of the first pivoting support; thereby enabling the endoscope to rotate around an insertion point into a body of a subject in at least two orthogonal axes;
    iii. at least one controller attached to either the first pivoting support or the second pivoting support

  b. pivotally attaching the at least one first pivoting support to the endoscope;
  c. positioning the at least one second pivoting support such that at least one axis of rotation of the at least one second pivoting support is substantially orthogonal to at least one axis of rotation of the at least one the first pivoting support;
  d. attaching the at least one controller to at least one of the at least one first pivoting support and the at least one second pivoting support;
  e. maneuvering the endoscope about the penetration point, thereby enabling maneuvering of the endoscope while maintaining alignment of the endoscope about the penetration point.

It is another object of the invention to disclose the method as defined above, comprising a further step of adapting at least one of the pivoting supports to moderate the pivoting of the endoscope around either the first axis of rotation or the second axis of rotation.

It is another object of the invention to disclose the method as defined above, comprising a further step of providing at least one connecting means in connection with the endoscope and at least one of the pivoting supports.

It is another object of the invention to disclose the method as defined above, comprising a further step of adapting at least one of the pivoting supports to constantly apply a counter torque to oppose the torque induced by the endoscope and the connecting means.

It is another object of the invention to disclose the method as defined above, comprising a further step of varying the counter torque as the torque varies, thereby enabling a constant dynamic equilibrium between the endoscope and at least one of the first pivoting support and the second pivoting support.

It is another object of the invention to disclose the method as defined above, comprising a further step of adapting the first pivoting support to enable the endoscope to pivot around one first axis of rotation and adapting the second pivoting support to enable the endoscope to pivot around a second and third axis of rotation, each of the first axis of rotation, the second axis of rotation and the third axis of rotation being substantially perpendicular to the other two axes of rotation.

It is another object of the invention to disclose the method as defined above, comprising a further step of providing the controller with a damping mechanism adapted to prevent oscillation of the system.

It is another object of the invention to disclose the method as defined above, comprising a further step of providing the controller with at least one of an active mechanism and a passive mechanism to provide the dynamic equilibrium.

It is another object of the invention to disclose the method as defined above, comprising a further step of providing the controller with at least one of a group consisting of: a motor and a spring mechanism to provide the dynamic equilibrium.

It is another object of the invention to disclose the method as defined above, comprising a further step of increasing the torque applied by the controller as the angle between the vertical and the endoscope increases.

It is another object of the invention to disclose the method as defined above, comprising a further step of enabling the control to be activated by at least one of a group consisting of: the control is activated at all times, the control is activated when the endoscope's angle is greater than a predetermined value, and the control is activated when the torque on the endoscope is greater than a predetermined value.

It is another object of the invention to disclose the method as defined above, comprising a further step of providing the system with an automatic assistance in communication with the endoscope.

It is another object of the invention to disclose the method as defined above, comprising a further step of adapting the automatic assistance to maneuver the endoscope.

It is another object of the invention to disclose the method as defined above, comprising a further step of providing the system with an alignment mechanism.

It is another object of the invention to disclose the method as defined above, comprising a further step of adapting the alignment mechanism to instruct the automatic assistance to align the endoscope.

It is another object of the invention to disclose the method as defined above, further comprising steps of:

a. providing a system comprising:

i. a first mechanism, comprising:

a) at least one first transmission means **101**; first transmission means **101** defines a first plane; first transmission means **101** is characterized by a first axis of rotation; the first axis of rotation is substantially orthogonal to the first plane;
b) at least one second transmission means **102**; second transmission means **102** defines a second plane; second transmission means **102** defines a second axis of rotation; the second axis of rotation is substantially orthogonal to the second plane; second transmission means **102** is rotatably connected to first transmission means **101**; where the first plane is substantially orthogonal to second plane; and
c) at least one first means **106** adapted to rotate first transmission means **101** around the first axis of rotation;

ii. a second mechanism, comprising:

a) at least one third transmission means **103**; third transmission means **103** defines a third plane; third transmission means **103** is characterized by a third axis of rotation; the third axis of rotation is substantially orthogonal to the third plane;
b) at least one fourth transmission means **104**; fourth transmission means **104** defines a fourth plane; fourth transmission means **104** defines a fourth axis of rotation; the fourth axis of rotation is substantially orthogonal to the fourth plane; the fourth transmission means **104** is rotatably connected to third transmission means **103**; the fourth plane is substantially orthogonal to the third plane;
c) at least one fifth transmission means **105**; fifth transmission means **105** defines a fifth plane; fifth transmission means **105** defines a fifth axis of rotation; the fifth axis of rotation is substantially orthogonal to the fifth plane; fifth transmission means **105** is rotatably connected to fourth transmission means 104; the fifth plane is substantially orthogonal to the fourth plane;
d) at least one second means **107** adapted to rotate third transmission means **103** around the third axis of rotation;

b. positioning first transmission means **101** orthogonal to second transmission means **102**; the positioning enables transmission of rotation between first transmission means **101** and second transmission means **102**;
c. positioning third transmission means **103** orthogonal to fourth transmission means **104**; the positioning enables transmission of rotation between third transmission means **103** and fourth transmission means **104**.
d. positioning fourth transmission means **104** orthogonal to fifth transmission means **105**; the positioning enables transmission of rotation between fourth transmission means **104** and fifth transmission means **105**.
e. coupling second transmission means **102** to the endoscope and fifth transmission means **105** to the endoscope; the coupling enables rotation of the endoscope proportional to rotation of second transmission means **102** and fifth transmission means **105**; and,
f. maneuvering the endoscope in at least two degrees of freedom (DOF); maneuvering of the endoscope in the at least two degrees of freedom are in second axis of rotation **141** and in fifth axis of rotation **142**;

wherein maneuvering in a first DOF of the at least two DOF is performed by a step of rotating first transmission means **101** thereby transmitting rotation to the endoscope; wherein maneuvering in a second DOF of at least two DOF is performed by a step of rotating third transmission means **103** thereby transmitting rotation to the endoscope.

It is another object of the invention to disclose the method as defined above, further comprising a step of defining an angle A between second axis of rotation **141** and fifth axis of rotation **142;** angle A is in the range of about 0 degrees to about 180 degrees.

It is another object of the invention to disclose the method as defined above, further comprising steps of

    a. providing at least one zoom mechanism **115;** and
    b. maneuvering the endoscope along the main longitudinal axis of the same.

It is another object of the invention to disclose the method as defined above, further comprising a step of selecting at least one of the first and second pivoting supports (**113** and **114)** to be a gimbal.

It is another object of the invention to disclose the method as defined above, further comprising a steps of

    a. selecting first transmission means **101,** second transmission means **102,** third transmission means **103,** fourth transmission means **104,** and fifth transmission means **105** from a group consisting of gearwheels, wheels, crown gears, bevel gears, spur gears, belts, and any combinations thereof and
    b. providing the system with attaching means adapted to reversibly couple the system to a hospital bed, the attaching means selected from a group consisting of mechanical means, magnetic means and any combination thereof.

It is another object of the invention to disclose the method as defined above, further comprising a step of selecting the mechanical means from a group consisting of a clip, a fastening element, tape, adhesive tape, a snap fastener, a button and any combination thereof.

It is another object of the invention to disclose the method as defined above, further comprising a step of providing, as the magnetic means, a magnetic device comprising at least one magnet and at least one selected from a group consisting of a ferromagnet and a paramagnet; where the magnet is attached to any member of a group consisting of: a hospital bed, the system, and any combination thereof, and at least one selected from a group consisting of a ferromagnet and a paramagnet is attached to at least one member of a group consisting of: a hospital bed, the system, and any combination thereof.

It is another object of the invention to disclose the method as defined above, further comprising a step of defining an angle $\theta$ for rotation in said second plane, the angle $\theta$ to vary between about 0 and about 360 degrees, preferably between about 0 and about 160 degrees, when the system is in its automatic configuration or in its manual configuration.

It is another object of the invention to disclose the method as defined above, further comprising a step of defining an angle $\psi$ for rotation in the fifth plane, the angle $\psi$ to vary between about 0 and about 360 degrees, preferably between about 0 and about 140 degrees, when the system is in its automatic configuration or in its manual configuration.

It is another object of the invention to disclose the method as defined above, further comprising a step of additionally providing the system with a quick release handle adapted to disassemble the endoscope from the system when the system is in its automatic configuration or in its manual configuration.

It is another object of the invention to disclose the method as defined above, further comprising a step of providing the first mechanism with locking means adapted to maintain in a predetermined orientation upon power failure at least one selected from a group consisting of: the first transmission means, the second transmission means and any combination thereof; and to prevent any rotational movement of the same upon power failure.

It is another object of the invention to disclose the method as defined above, further comprising a step of providing the second mechanism additionally with locking means adapted to maintain in a predetermined orientation upon power failure at least one selected from a group consisting of: the third transmission means, the fourth transmission means, the fifth transmission means, and any combination thereof; and to prevent any rotational movement of the same upon power failure.

It is another object of the invention to disclose the method as defined above, further comprising a step of providing at least one manual override system (MOS), adapted upon activation of the same to switch reversibly between a manual configuration, in which the endoscope is moved manually by the operator and an automatic configuration, in which the endoscope is moved automatically by the system and optionally further comprising a step of enabling the same to switch reversibly to a third configuration, in which the endoscope is moved wholly manually by an endoscope assistant.

It is another object of the invention to disclose the method as defined above, further comprising a step of providing at least one joystick, in communication with the endoscope.

It is another object of the invention to disclose the method as defined above, further comprising a step of providing activation means adapted to activate at least one of a group consisting of the system, the joystick and any combination thereof.

It is another object of the invention to disclose the method as defined above, further comprising a step of enabling the joystick to be worn by the joystick user.

It is another object of the invention to disclose the method as defined above, further comprising a step of enabling the activation means to be worn by the MOS user.

It is another object of the invention to disclose the method as defined above, further comprising a step of selecting the activation means from a group consisting of a pressable button, a rotatable knob, a knob, and any combination thereof.

It is another object of the invention to disclose the method as defined above, further comprising a step of enabling the MOS to rotate in the angles $\psi$ and $\theta$.

It is another object of the invention to disclose the method as defined above, further comprising a step of defining angles $\alpha$ and $\beta$ such that the endoscope moves in angular direction $\theta$ when the joystick is moved in direction $\alpha$, and the endoscope moves in angular direction $\psi$ when the joystick is moved in direction $\beta$. It is another object of the invention to disclose the method as defined above, wherein movement of the joystick in a direction selected from a group consisting of $\alpha$, $\beta$ and any combination thereof, is proportional to movement of the endoscope in angular directions $\psi$, $\theta$ and any combination thereof.

It is another object of the invention to disclose the method as defined above, further comprising a step of providing the MOS with means for controlling the endoscope motion, adapted to moderate angular velocity in the $\theta$ and $\psi$ directions.

It is another object of the invention to disclose the method as defined above, further comprising a step of providing the MOS with $n$ sensors, where $n$ is an integer greater than or equal to one.

It is another object of the invention to disclose the method as defined above, further comprising a step of selecting the sensors from of a group consisting of: motion sensors, heat sensors, electric sensors, sound sensors, pressure sensors, optical sensors and any combination thereof.

It is another object of the invention to disclose the method as defined above, further comprising a step selected from at least one of the following: activating the $n$ sensors in case of power failure and activating the $n$ sensors when the system is connected to power.

It is another object of the invention to disclose the method as defined above, further comprising step of detecting the motion of the joystick with motion sensors.

It is another object of the invention to disclose the method as defined above, further comprising a step of using the motion detection of the joystick for deactivation of the motion of the endoscope if the motion's speed is above a predetermined threshold.

It is another object of the invention to disclose the method as defined above, further comprising a step of characterizing the joystick by an external surface.

It is another object of the invention to disclose the method as defined above, further comprising a step of operating the motion sensors to detect motion upon the external surface.

It is another object of the invention to disclose the method as defined above, further comprising a step of operating the endoscope according to the motion upon the external surface.

It is another object of the invention to disclose the method as defined above, further comprising a step of deactivation of the motion of the endoscope when the motion's speed along the joystick is above a predetermined threshold.

It is another object of the invention to disclose the method as defined above, further comprising a step of adapting the heat sensors to sense temperatures in the range of about 35 to about 42 degrees.

It is another object of the invention to disclose the method as defined above, further comprising a step of enabling the activation of the MOS when the heat sensors sense that the temperature is in the range of about 35 to about 42 degrees.

It is another object of the invention to disclose the method as defined above, further comprising a step of adapting the heat sensors to provide at least one thermal image, where the heat sensors are coupled to a processing unit adapted to provide the endoscope user with the thermal image.

It is another object of the invention to disclose the method as defined above, further comprising a step of adapting the electric sensors to sense power failure.

It is another object of the invention to disclose the method as defined above, further comprising a step of adapting the electric sensors to sense electrical conductivity of human body.

It is another object of the invention to disclose the method as defined above, further comprising a step of enabling the activation of the MOS upon sensing the human body's conductivity by electric sensors.

It is another object of the invention to disclose the method as defined above, further comprising a step of adapting the sound sensors to sense predetermined sound patterns.

It is another object of the invention to disclose the method as defined above, further comprising a step of operating the endoscope according to predetermined sound patterns sensed by the sound sensors.

It is another object of the invention to disclose the method as defined above, further comprising a step of adapting the pressure sensors to sense pressure applied to the MOS.

It is another object of the invention to disclose the method as defined above, further comprising a step selected from at least one of a group consisting of: activating the MOS when the pressure sensed by the pressure sensors is above a predetermined threshold and de-activating the MOS when the pressure sensed by the pressure sensors is below a predetermined threshold.

It is another object of the invention to disclose the method as defined above, further comprising a step of adapting the optical sensors to sense visual changes according to predetermined visual patterns.

It is another object of the invention to disclose the method as defined above, further comprising a step of operating said endoscope according to predetermined visual patterns detected by said sensors.

It is another object of the invention to disclose the method as defined above, further comprising a step of enabling the activation of said MOS upon at least one condition selected from a group consisting of: analysis of said thermal image by said processing unit and detection of a human hand, said electric sensors sense said human body conductivity, said sound sensors sense said predetermined sound patterns, and according to said predetermined visual patterns detected by said optical sensors.

It is another object of the invention to disclose the method as defined above, further comprising a step of altering the activation state of said MOS under at least one condition selected from a group consisting of: when said pressure sensed by said pressure sensors is above a predetermined threshold said MOS is activated, and when said pressure sensed by said pressure sensors is below a predetermined threshold, said MOS is de-activated.

## BRIEF DESCRIPTION OF THE FIGURES

In order to understand the invention and to see how it may be implemented in practice, a few preferred embodiments will now be described, by way of non-limiting example only, with reference to be accompanying drawings, in which

**Fig. 1** presents a system for maneuvering an endoscope;

**Fig. 2a** and **2b** shows two configurations of the system for maneuvering an endoscope attached to a rotating means;

**Figs 3a-c, 4a-b** and **5a-b** demonstrate more configurations of a system for maneuvering an endoscope;

**Fig. 6** presents the axes of rotation for rotation of the endoscope about the joints.

**Fig. 7** illustrates the forces and torques on an endoscope attached to a pivoting support;

**Fig. 8** schematically illustrates the normal force on the penetration point due to the endoscope;

**Fig. 9a-b** and **10** presents a pivoting support with a controller;

**Fig. 11** presents a system for maneuvering an endoscope with pivoting supports comprising controllers;

**Fig. 12** presents a pivoting support and controller with transparent cover;

**Fig. 13** presents the pivoting support, showing a connector adapted to join the pivoting supports to the connecting arm linking the pivoting supports to the endoscope;

**Figs. 14-17** show different configurations for the motors of a system for maneuvering an endoscope;

**Fig. 18** presents a configuration of system with a hospital bed and an endoscope;

**Fig. 19** depicts another configuration of the system in an operating room, with an emphasis on movement range;

## DETAILED DESCRIPTION OF THE INVENTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

A system for maneuvering an endoscope, comprising:

a. at least one first pivoting support adapted to be pivotally attached to said endoscope; said pivoting support adapted to enable said endoscope to pivot around at least one first axis of rotation;

b. at least one second pivoting support in communication with said at least one first pivoting support, said second pivoting support adapted to rotate around at least one axis being substantially orthogonal to said first axis of rotation independently of said first pivoting support; thereby enabling said endoscope to rotate around an insertion point into a body of a subject in at least two orthogonal axes;

c. at least one controller attached to either said first pivoting support or said second pivoting support,

wherein said controller is adapted to provide a constant dynamic equilibrium between said endoscope and at least one of said first pivoting support or said second pivoting support.

The present invention discloses the system further comprising:

a. a first mechanism, comprising:

i. at least one first transmission means; the first transmission means defines a first plane and is characterized by a first axis of rotation which is substantially orthogonal to the first plane;

ii. at least one second transmission means; the second transmission means defines a second plane and second axis of rotation; the second axis of rotation is substantially orthogonal to the second plane; and the second transmission means is rotatably connected to the first transmission means; where the first plane is substantially orthogonal to the second plane; and

iii. at least one first means adapted to rotate the first transmission means around the first axis of rotation;

where the first transmission means transmits rotation to the second transmission means; and,

b. a second mechanism, comprising:

i. at least one third transmission means which defines a third plane and is characterized by a third axis of rotation; the third axis of rotation is substantially orthogonal to the third plane;

ii. at least one fourth transmission means which defines a fourth plane and s fourth axis of rotation; the fourth axis of rotation is substantially orthogonal to the fourth plane; and the fourth transmission means is rotatably connected to the third transmission means; where the fourth plane is substantially orthogonal to the third plane;

iii. at least one fifth transmission means which defines a fifth plane and a fifth axis of rotation; the fifth axis of rotation is substantially orthogonal to the fifth plane; the fifth transmission means is rotatably connected to the fourth transmission means and is substantially orthogonal to the fourth plane;

iv. at least one second means adapted to rotate the third transmission means around the third axis of rotation;

where the third transmission means transmits rotation to the fourth transmission means; the fourth transmission means transmits rotation to the fifth transmission means,

wherein the first mechanism and the second mechanism are adapted to rotate the endoscope around at least one second axis of rotation substantially orthogonal to the second plane; and around at least one fifth axis of rotation substantially orthogonal to the fifth plane, such that the second axis of rotation and the fifth axis of rotation are positioned at an angle A relative to each other.

The present invention additionally provides a method for maneuvering an endoscope comprising steps of:

a. providing a system comprising:

i. a first mechanism, comprising:

a) at least one first transmission means which defines a first plane; and is characterized by a first axis of rotation; the first axis of rotation is substantially orthogonal to the first plane;

b) at least one second transmission means which defines a second plane and a second axis of rotation; the second axis of rotation is substantially orthogonal to the second plane; the second transmission means is rotatably connected to the first transmission means and the first plane is substantially orthogonal to the second plane; and

c) at least one first means adapted to rotate the first transmission means around the first axis of rotation;

ii. a second mechanism, comprising:

a) at least one third transmission means which defines a third plane; the third transmission means is characterized by a third axis of rotation; the third axis of rotation is substantially orthogonal to the third plane;

b) at least one fourth transmission means which defines a fourth plane and a fourth axis of rotation; the fourth axis of rotation is substantially orthogonal to the fourth plane; the fourth transmission means is rotatably connected to the third transmission means such that the fourth plane is substantially orthogonal to the third plane;

c) at least one fifth transmission means which defines a fifth plane and a fifth axis of rotation; the fifth axis of rotation is substantially orthogonal to the fifth plane; the fifth transmission means is rotatably connected to the fourth transmission means such that the fifth plane is substantially orthogonal to the fourth plane; and

d) at least one second means adapted to rotate the third transmission means around the third axis of rotation;

b. positioning the first transmission means orthogonal to the second transmission means; where this positioning enables transmission of rotation between the first transmission means and the second transmission means;

c. positioning the third transmission means orthogonal to the fourth transmission means; where this positioning enables transmission of rotation between the third transmission means and the fourth transmission means;

d. positioning the fourth transmission means orthogonal to the fifth transmission means; where this positioning enables transmission of rotation between the fourth transmission means and the fifth transmission means;

e. coupling the second transmission means to the endoscope and the fifth transmission means to the endoscope; where the coupling enables rotation of the endoscope proportional to the rotation of the second transmission means and to the fifth transmission means; and

f. maneuvering the endoscope in at least two degrees of freedom (DOF);

wherein maneuvering in a first DOF of the at least two DOFs is performed by a step of rotating the first transmission means thereby transmitting rotation to the endoscope; wherein maneuvering in a second DOF of the at least two DOFs is performed by a step of rotating the third transmission means thereby transmitting rotation to the endoscope.

The term **'endoscope'** refers hereinafter to any means adapted for looking inside the body for medical reasons. This may be any instrument used to examine the interior of a hollow organ or cavity of the body. The endoscope may also refer to any kind of a laparascope.

The term **'spatial position'** refers hereinafter to a predetermined spatial location and/or orientation of an object (e.g., the spatial location of the endoscope, the angular orientation of the endoscope, and any combination thereof).

The term **"degrees of freedom" (DOF)** refers hereinafter to a set of independent displacements that specify completely the displaced position of the endoscope or laparoscope as defined above. In three dimensional space, there are six DOFs, three DOFs of linear displacement and three rotational DOFs, namely, moving up and down, moving left and right, moving forward and backward, tilting up and down, turning left and right, tilting side to side. According to some embodiments, the present invention refers to a system essentially comprising means for at least seven DOF selected from any of those that will be described hereinafter.

The term **"about"** refers hereinafter to a range of +-25% of the discussed quantity.

The term **"operator"** refers hereinafter to a user of the system. Examples of operators are the surgeon, the operating medical assistant, the surgeon's colleagues, etc.

The term **'automated assistant'** refers hereinafter to any mechanical device (including but not limited to a robotic device) that can maneuver and control the position of a surgical or endoscopic instrument, and that can in addition be adapted to receive commands from a remote source.

The term **'provide'** refers hereinafter to any process (visual, tactile, or auditory) by which an instrument, computer, controller, or any other mechanical or electronic device can report the results of a calculation or other operation to a human operator.

The term **'automatic'** or **'automatically'** refers to any process or action that proceeds without the necessity of direct intervention or action on the part of a human being.

The term **'manual'** or **'manually'** refers to any process or action necessitating direct intervention or action on the part of a human being. For a non-limiting example, an endoscope moved by a motor is under manual control when a human operator instructs the motor as to the movements of the endoscope. Such instructions can be, for example, via a movements of a joystick or via voice commands.

The term **'wholly manual'** or **'wholly manually'** refers to any process or action where the process or action is carried out by a human being without mechanical intervention or assistance. For example, an endoscope assistant can provide wholly manual control of an endoscope, maneuvering it directly and without mechanical assistance in response to, for example, voice commands by a physician.

The term 'motor' refers hereinafter to anything that produces or imparts motion. A motor includes, but is not limited to, an engine, an electric motor, an induction motor, a reciprocating engine, a Wankel engine, a hydraulic engine, devices employing shape memory alloys, and traction engines.

The term 'transmission means' refers hereinafter to anything that transmits movement from a motor to an object to be moved. Transmission means include, but are not limited to, gears, pulleys, gearwheels, wheels, crown gears, bevel gears, spur gears, belts, and any combination thereof,

The term **'dynamic equilibrium'** refers hereinafter to maintaining the endoscope in a condition such that it the only effective torque on it is the torque deliberately applied by the system in order to rotate it. Any torques induced by the weight of the endoscope head are counterbalanced by opposing torques. Said opposing torques can be applied by springs, motors, counterweights or any other means known in the art.

The term **'counter torque'** refers hereinafter to any torque applied to the endoscope and the connecting means adapted to connect the endoscope to at least one of the pivoting supports which counteracts the torques caused by the endoscope and the connecting means.

The term **'damping mechanism'** refers hereinafter to any mechanism which does at least one of the following:

reduces oscillatory motion of the endoscope head; and reduces acceleration of the endoscope head, especially at the beginning and the end of the motion.

The term **'passive mechanism'** refers hereinafter to a mechanism which depends only on the physical properties of the mechanism itself in order to operate. A non-limiting example of a passive mechanism is a spring.

The term **'active mechanism'** refers hereinafter to a mechanism which requires external input, such as, but not limited to, a source of power, input from sensors or a control mechanism in order to operate. An active mechanism can have more flexibility than a passive mechanism but can also be more prone to unwanted side-effects such as oscillation. A non-limiting example of an active mechanism is a motor. The term **'the vertical'** refers hereinafter to the direction which is parallel to the direction of the acceleration due to gravity and points away from the earth.

The term **'angle between the vertical and the endoscope'** refers hereinafter to the angle between the vertical and the longitudinal axis of the endoscope. The angle is zero if the longitudinal axis of the endoscope is vertical and the angle is 90° if the longitudinal axis of the endoscope is horizontal.

The following abbreviations are used throughout the disclosure:

**DOF** refers to degree(s) of freedom;
**MOS** refers to manual override system;
**FTM** refers to first transmission means;
**STM** refers to second transmission means;
**TTM** refers to third transmission means;
**FOTM** refers to fourth transmission means; and,
**FTTM** refers to fifth transmission means.

One of the main objects of the present invention is to disclose an endoscope maneuvering device in which the working angle of the endoscope can be substantially small. Namely the physician would be able to maneuver the endoscope at angles which are tangent to the patient treated (namely about 0-30 degrees relative to the upper surface of the patient's treated organ).

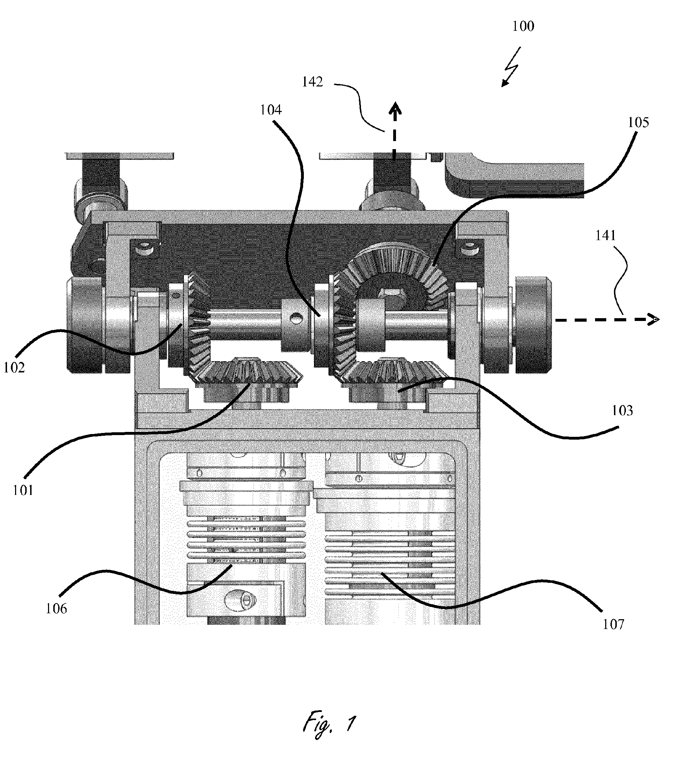

Reference is now made to Fig. **1,** which shows in a non-limiting manner, a first part **100** of a system for maneuvering an endoscope **200.**

The system comprises a first mechanism for maneuvering an endoscope in one **DOF.** The first mechanism comprises: (i) At least one first transmission means **(FTM) 101,** where the **FTM** is characterized by a first axis of rotation and a first plane substantially orthogonal to the first axis of rotation. (ii) At least one second transmission means **(STM) 102,** where the **STM** is characterized by a second axis of rotation and a second plane substantially orthogonal to the second axis of rotation **141.** Additionally, the **STM** is rotatably connected to the **FTM.** (iii) At least one first means **106** (especially a motor) adapted to rotate **FTM 101** around a first axis of rotation. The **FTM 101** transmits the rotation to the **STM 102.** Additionally, the system also comprises a second mechanism for maneuvering an endoscope **200** at a second **DOF.** The second mechanism comprises: (i) At least one third transmission means **(TTM) 103,** where the **TTM 103** is characterized by a third axis of rotation and a third plane substantially orthogonal to the third axis of rotation. (ii) At least one fourth transmission means **(FOTM) 104,** where the **FOTM** is characterized by a fourth plane, and a fourth axis of rotation substantially orthogonal to the fourth plane, and the FOTM is rotatably connected to the **TTM 103.** The connection is such that the fourth plane is substantially orthogonal to the third plane. (iii) At least one fifth transmission means **(FTTM) 105.** The **FTTM 105** defines a fifth plane and a fifth axis of rotation **142** substantially orthogonal to the fifth plane, and the **FTTM 105** is rotatably connected to the **FOTM 104.** The connection is such that the fifth plane is substantially orthogonal to the fourth plane. (iv) At least one second means **107** (especially a motor) adapted to rotate **TTM 103** around the third axis of rotation. The **TTM 103** transmits rotation to **FOTM 104,** the **FOTM 104** than transmits rotation to the **FTTM 105.** The system then maneuvers the endoscope 200 by adapting the first mechanism to rotate the endoscope **200** in one **DOF** substantially orthogonal to the second plane *(i.e.* second axis of rotation **141),** and adapting the second mechanism to rotate the endoscope **200** in a second **DOF** substantially orthogonal to the fifth plane *(i.e.* fifth axis of rotation **142).** The two **DOF** define two axes of rotation with angle A between them. The angle A is in the range of 0 to 180 degrees.

In some embodiments, at least some of the **FTM, STM, TTM, FOTM** and **FTTM** are coaxial, so that at least two transmission means share the same axis of rotation. A non-limiting example of coaxial transmission means would be transmission means linked by a universal joint, where the two transmission means transmit rotations in two perpendicular directions.

In other embodiments, at least one of the **FTM, STM, TTM, FOTM** and **FTTM** comprises a plurality of coaxial transmission means.

In the best embodiment, both the first means **106** and the second means **107** are static, in that both are mounted in fixed positions. The system has been designed so that the transmission means, especially **FOTM** and **FTTM,** can be driven by said means (106 and 107) with the means 106 and 107 in fixed positions.

This reduces the number of moving parts in the system, thereby improving its reliability. It is also more difficult for the system to get out of alignment, and for the gears to jam, as the main between alignments between the **FTM, STM, TTM, FOTM** and **FTTM** are fixed at the time of manufacture and do not vary during use. It also enables the system to be more compact, as there is no need to allow space for a mechanism to move within.

In preferred embodiments of the current invention, the second plane defines in a non-limiting manner angle $\theta$ and the fifth plane defines in a non-limiting manner the angle $\psi$. The angle $\theta$ varies between about 0 and about 360 degrees, preferably between about 0 and about 160 degrees, when system **100** is in automatic configuration or in manual configuration. Additionally, angle $\psi$ varies between about 0 and about 360 degrees, preferably between about 0 and about 140 degrees, when system **100** is in automatic configuration or in manual configuration.

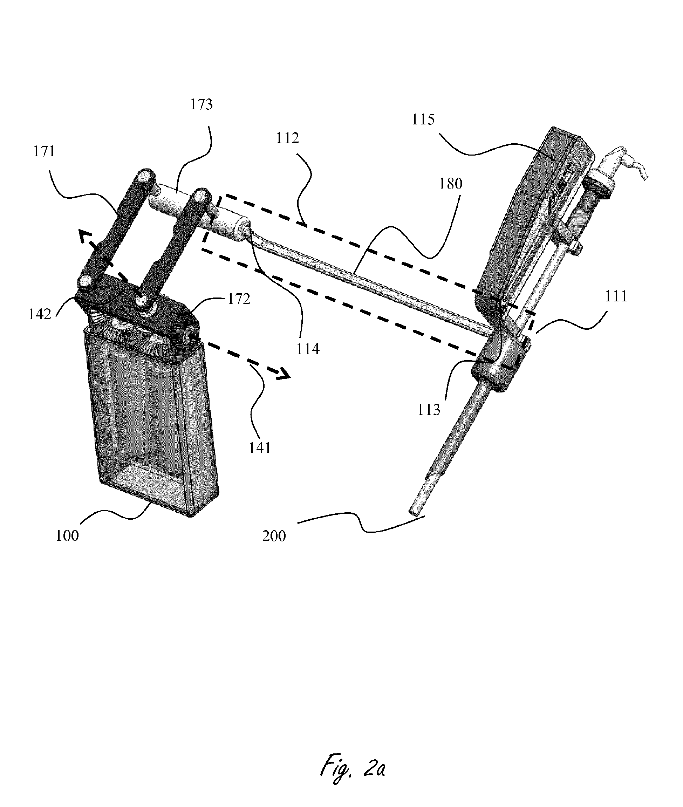

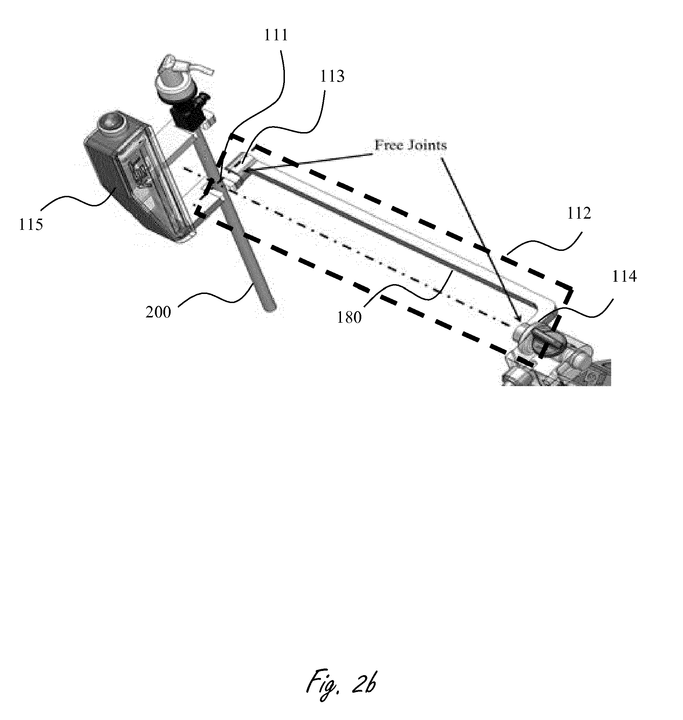

Reference is now made to Figs. **2a** and **2b,** which present in a non-limiting manner a rotating means in communication with the first mechanism and the second mechanism. The rotating means comprises (i) at least one pivoting support **111** adapted to be pivotally attached to endoscope **200,** pivoting support **111** is adapted to enable endoscope **200** to pivot around pivoting support **111;** (ii) least one third mechanism **112** for rotating the endoscope **200** independently around two orthogonal axes; the third mechanism **112** mechanically connected to pivoting support **111,** thereby enabling endoscope **200** to rotate around an insertion point in the body of a subject.

The third mechanism **112** (will also be referred to as connecting arm **612**) comprising at least one pivoting support **111;** and at least one second joint **114** (namely, a second pivoting support or gimbal) in communication with first pivoting support **111** and coupled to a mechanism selected from a group consisting of the first mechanism, the second mechanism and any combination thereof.

Said second pivoting support 114 is coupled to said first pivoting support 111 by means of a rod, an arm, *n* joints (n being an integer number greater than or equals to 1).

Each of the joints is adapted to provide rotation to pivoting support **111** in at least one of the orthogonal axes; wherein second joint **114** is located at a predetermined distance **180** from first joint **113.**

In the best embodiment, gimbals or other joint mechanisms at first joint **113** and second joint **114** enable endoscope **200** to pivot about its insertion point in the body of the patient without applying force on the patient at the insertion point, especially if the line of application of force to move the endoscope is not completely collinear with the axis of the endoscope.

The pair of gimbals or other joint mechanisms at joints **113** and **114** enable sufficient flexibility that the insertion point can remain fixed without the application of force by the body of the patient.

It should be emphasized that the addition of the pair of joint mechanisms **113** and **114** ensure that no force is applied on the penetration point when the system's center of movement is misaligned with the penetration point.

It should be emphasized that according to a preferred embodiment of the present invention, joint mechanisms **113** and **114** are gimbals.

In one embodiment, each of joint mechanisms **113** and **114** has one DOF, preferably rotations about axes substantially perpendicular to each other. A non-limiting example of such a pair of rotations is shown in **Figs. 2a** and **2b,** where joint mechanism **114** rotates about an axis parallel to second axis of rotation **141** and joint mechanism **113** rotates about an axis of rotation perpendicular to this and parallel to the base of zoom mechanism **115.** However, this embodiment is less preferred because of the possibility of pressure on the penetration point in a direction perpendicular to the third axis of rotation.

In preferred embodiments, one of joint mechanism **113** and joint mechanism **114** is enabled to rotate about two substantially perpendicular axes of rotation, while the other joint mechanism rotates about a third axis of rotation, substantially perpendicular to both of the other axes of rotation. In some variants, joint mechanism **113** can rotate about two substantially perpendicular axes of rotation, while joint mechanism **114** rotates about the third axis of rotation, substantially perpendicular to the other two, thereby enabling rotation of the endoscope about all three axes of rotation and preventing pressure on the penetration point. In other variants, joint mechanism **114** can rotate about two perpendicular axes of rotation, while joint mechanism **113** rotates about the third axis of rotation, substantially perpendicular to the other two, thereby enabling rotation of the endoscope about all three axes of rotation and preventing pressure on the penetration point.

It should be further emphasized that while moving (rotating) the first mechanism (which comprises the first transmission means **101** and the second transmission means **102),** the second mechanism (which comprises the third transmission means **103,** the fourth transmission means **104** and the fifth transmission means **105)** is moved (rotated) in the opposite direction and vice versa. Such reverse movement is highly important to compensate any unwanted /parasitic movement that would be created when moving only one mechanism.

Zoom mechanism **115** is connected to endoscope **111.**

Reference is now made again to Fig. **2a** which demonstrates in a non-limiting manner another object of the present invention.

In this figure a mechanism with sides forming a parallelogram for transforming the rotational movement to the endoscope is presented. As can be seen in the figure the parallelogram comprises rods **171** and **172.** Rod **172** is adapted to transform rotation around the second axis of rotation **141** to the endoscope and two rods **171** are adapted to transform rotation around fifth axis of rotation **142,** wherein the two rods **171** are connected to rod **172.**

Rods **171, 172** and **173** form a parallelogram.

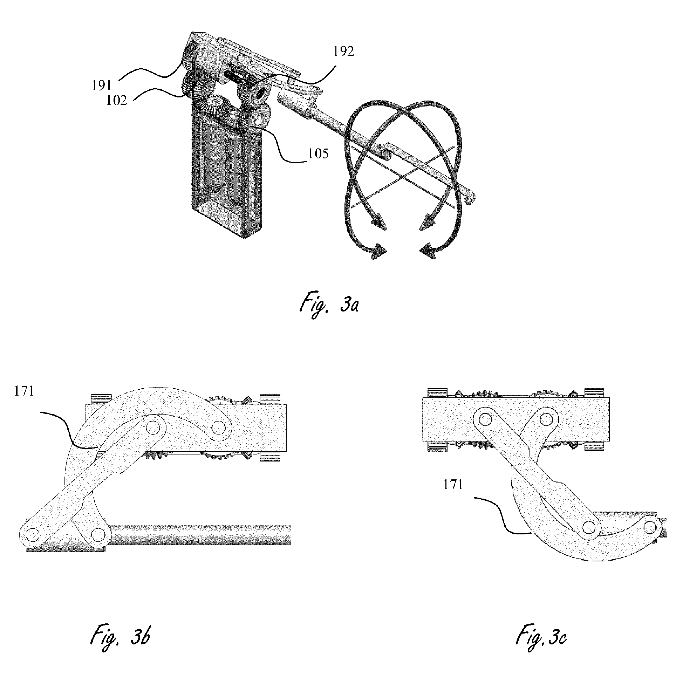

Reference is now made to Fig. **3a, 3b** and **3c,** which illustrate in a non-limiting manner a parallelogram adapted to communicate between the different transmission means and the endoscope.

In the figures, the above mentioned parallelogram is characterized by having at least one non-straight rib. As can be seen, at least one rib is shaped as an arced rod.

It is within the core concept of the present invention to provide an arc shaped parallelogram, namely one characterized by at least one arc-shaped side. According to one embodiment of the present invention, the arc shaped parallelogram provides the endoscope with a wide range of angular movements and maneuverability when compared to a parallelogram with all sides straight, a non-arc shaped parallelogram.

In addition, Fig. **3a** describes two additional (and 'intermediate') means **191, 192** constructed upon second transmission means **102** and fifth transmission means.

It is within the core concept of the present invention to provide the first and second mechanisms having at least one first transmission **101** (but there could be several interconnected transmissions); at least one second transmission **102** (but there could be several communicating transmissions); at least one third transmission **103** (but there could be several communicating transmissions); at least one second fourth transmission **104** (but there could be several communicating transmissions); at least one fifth transmission **105** (but there could be several communicating transmissions) and any combination thereof.

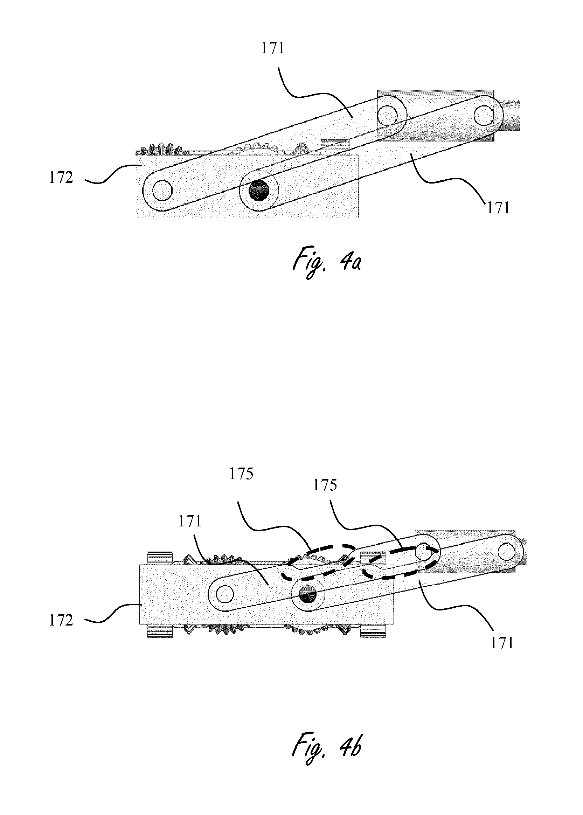

Reference is now made to Figs. **4a,** and **4b,** which illustrate in a non-limiting manner another embodiment of the parallelogram described above. The figures illustrate an embodiment in which ribs **171** comprise a dent (i.e., groove) **175** and an embodiment in which ribs **171** are not provided with dent **175.**

Fig. **4a** demonstrates the failure of rods **171** to achieve a maximum 180 degrees angle with respect to rod 172. Such a failure is the result of the collision of ribs **171** with each other.

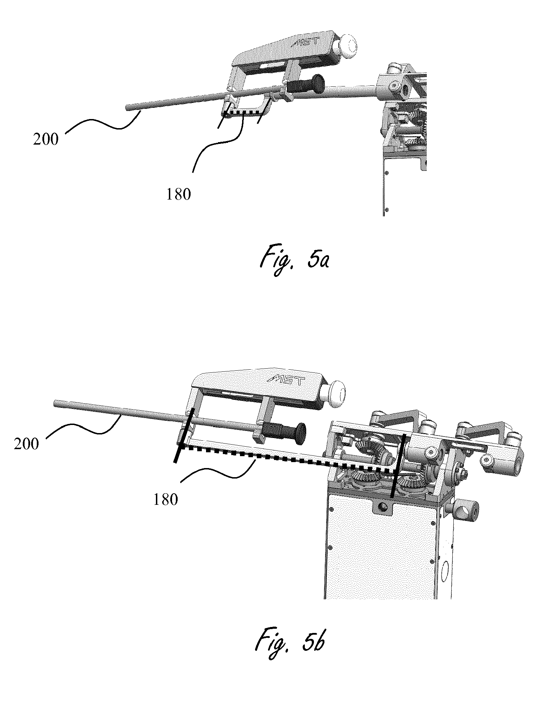

In Fig. **4b** a solution is suggested in a form of a dent **175** in rods **171** which enables a larger angular movement of ribs **171.** By providing the dent (i.e., groove) **175,** a further angular movement is achieved. Reference is now made to Figs. **5a** and **5b,** which illustrate in a non-limiting manner the pre-determined distance **180** at 2 different lengths.

Fig. **5a** illustrates a relatively small predetermined distance **180,** such that the same limits the range of motion of the endoscope **200;** Fig. **5b** illustrates a larger predetermined distance **180,** such that the same enables the full range of motion of the endoscope **200.**

In other embodiments of the present invention, system **100** is characterized in a non-limiting manner by at least two configurations: an automatic configuration, in which system **100** is motorized; and a manual configuration in which system **100** is maneuvered manually by an endoscope user via a manual control mechanism, preferably a joystick. The system can also be characterized by a third configuration, a wholly manual configuration, in which a human endoscope assistant maneuvers the endoscope without mechanical assistance.

In another embodiment of the present invention, system **100** additionally comprises in a non-limiting manner a rotating means as described in Fig. **2** without a pivoting support such as **111.**

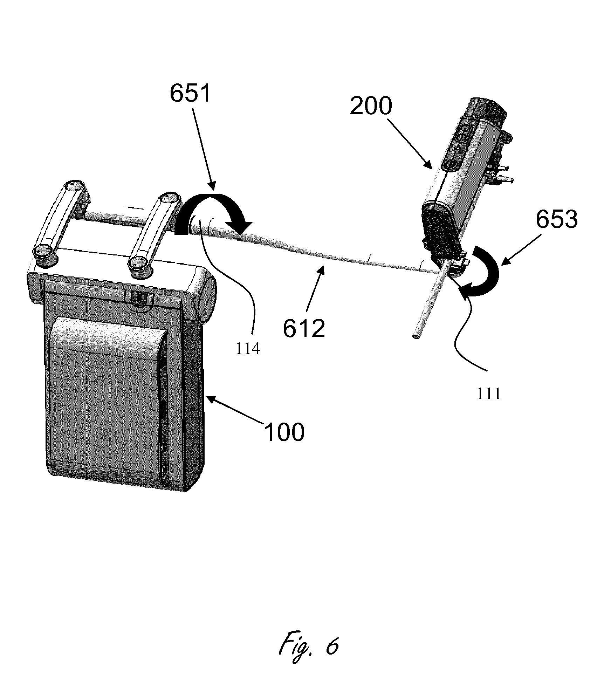

In reference to **Fig. 6,** an embodiment of the endoscope is shown where the third mechanism connecting the laparoscopic unit **(200)** to the control unit **(100)** is a connecting arm **(612).** Joint **113** connecting the endoscope, here a laparoscopic unit **(200),** to the connecting arm **(612)** allows a first passive DOF **(653)** and joint **114** (e.g., gimbal) connecting the control unit **(100)** to the connecting arm **(612)** allows a second passive DOF. These passive DOF reduce the forces applied by the laparoscopic unit on the penetration point.

It should be pointed that according to this embodiment, joint (or gimbal) **114** allows the connecting arm **612** (and thus, endoscope **200**) to rotate around said arm's **612** main longitudinal axis.

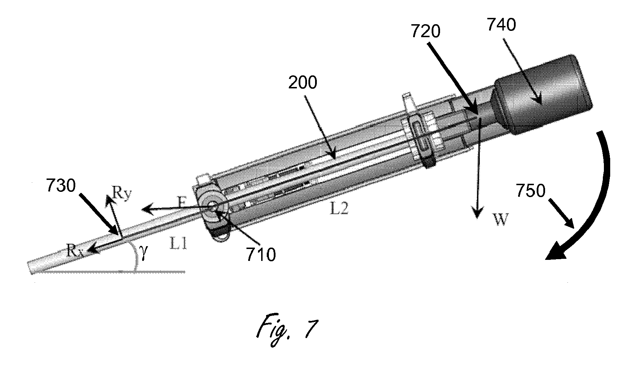

In reference to **Fig. 7,** a method of calculating the force on the penetration point is shown. An embodiment of the endoscope **(200)** is shown, comprising a laparoscopic unit, a laparoscopic camera unit **(740)** and a light cable (not shown). The laparoscopic unit **(200)** comprises a zoom mechanism (not shown) and is attached to the connecting arm **(612)** via joint **114,** (not shown) at gimbal point **710.** The penetration point **(730)** is also shown.

The weight of the endoscope 200 is W. Weight W, applied through the center of gravity of the unit **(720),** will tend to rotate the endoscope **200** about pivot point **710** in the direction of arrow **750,** with the magnitude of the torque being

$$\tau_W = \mathbf{L_2} \times \mathbf{W} = L_2 W \cos \alpha,$$

where $\alpha$ is the angle between the axis of the endoscope and the vertical. This will cause a counter-torque on the penetration point **730**

$$\tau_P = \mathbf{L_1} \times \mathbf{R} = L_1 R_y$$

When the system is in equilibrium, $\tau_W = \tau_P$, so that

$$R_y = L_2 W \cos \alpha / L_1 \qquad\qquad (1)$$

It should be noted that, in the above calculation, it is assumed that the force **F** applied by the control unit to the penetration point is small and has been neglected in these calculations.

As a non-limiting example, a typical laparoscopic unit has a weight of about 700 gm, while the laparoscopic camera unit, including its cable, weighs about 650 gm, for a total weight W of about 1350 gm. The distance L1 between the penetration point **730** and the pivoting point **710** is about 5 cm. The angle $\alpha$ varies between about 0 and about 90°, while $L_2$, the distance between the center of gravity **720** and the pivot point **710** varies between about 8 and about 13 cm.

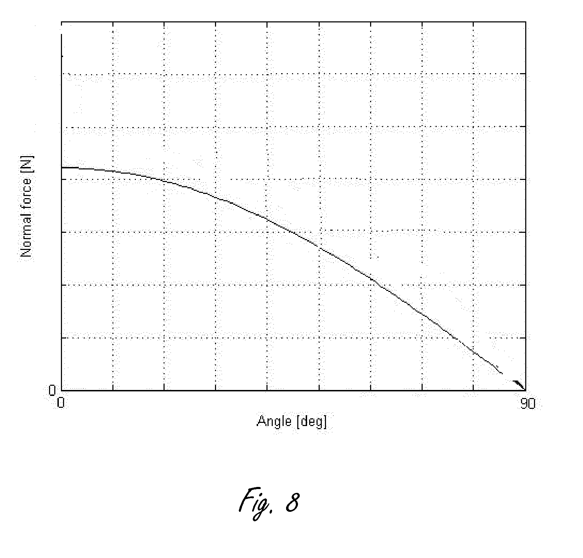

In reference **Fig. 8,** an exemplary plot is shown of the magnitude of the normal force $R_y$ on the pentration point as a function of angle $\alpha$ for different zoom positions $L_2$. The normal force $R_y$ is a maximum for the maximal zoom out position and a vertical endoscope (angle $\alpha = 90°$). The minimal force curve occurs for maximal zoom-in ($L_2$ a minimum).

The pressure on the penetration point depends on the force on the penetration point, $R_y$, and also on the area A over which it is applied, since $P = F/A = R_y/A$.

it should be pointed that the area over which the force is applied depends on the diameter of the trocar (D) and the thickness of the skin (t). The force is applied over the lower half-cylinder of skin in contact with the trocar. The area of skin in contact with the trocar is

$$A_c = (2 \pi r) \, t$$

where $2\pi r$ is the circumference of the cylinder and t is its height. Therefore, the area over which the pressure is applied is half that,

$$A = \pi \, r \, t$$

and the pressure on the penetration point is

$$P = R_y / A = R_y / \pi \, r \, t \qquad\qquad (2)$$

Basically, as is expected, when the endoscope is position vertically to the ground (floor) the center of mass of the same applies the maximum force (and thus, torque).

It should be pointed that in order to maintain control over the endoscope position and to minimize the effect of differing endoscope weights and angles on the controllability of the endoscope positioning system, at least one of joint mechanisms **113** and **114** includes a mechanism adapted to modify movement of the gimbal. In preferred embodiments, this mechanism to modify movement is a controller adapted to apply a torque to the joint mechanism, the torque being a function of the angle $\theta$, so that the endoscope **200** plus zoom mechanism **115** remains in dynamic equilibrium about at least one of axes **651** and **653**. The dynamic equilibrium is such that the torque $L_2 W \cos \alpha$ is substantially equal to the torque supplied by the controller.

The controller can supply torque to the system passively or actively, or a combination thereof. A passive means of supplying torque is one that requires no external input in order to operate; the action of a passive mechanism depends only on the physical properties of the mechanism itself. A non-limiting example of a passive mechanism is a device comprising a spring or springs.

An active means of supplying torque, on the other hand, requires external input, such as, but not limited to, a source of power, input from sensors or a control mechanism in order to operate. An active mechanism can have more flexibility than a passive mechanism but can also be more prone to unwanted side-effects such as oscillation. A non-limiting example of an active mechanism comprises a motor and at least one sensor.

A non-limiting example of a combined device comprises at least one spring with at least one motor.

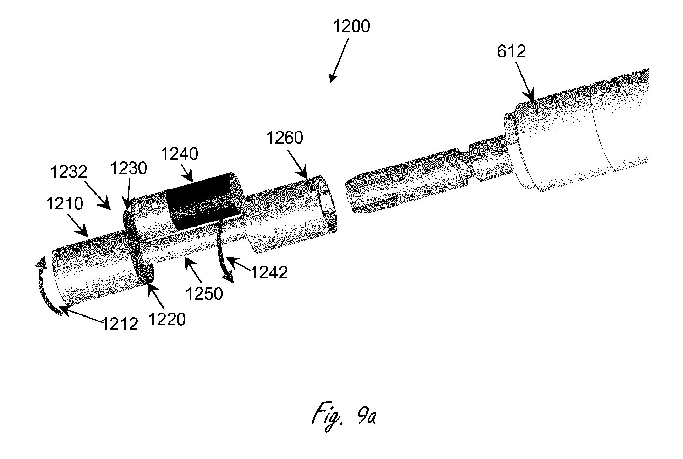

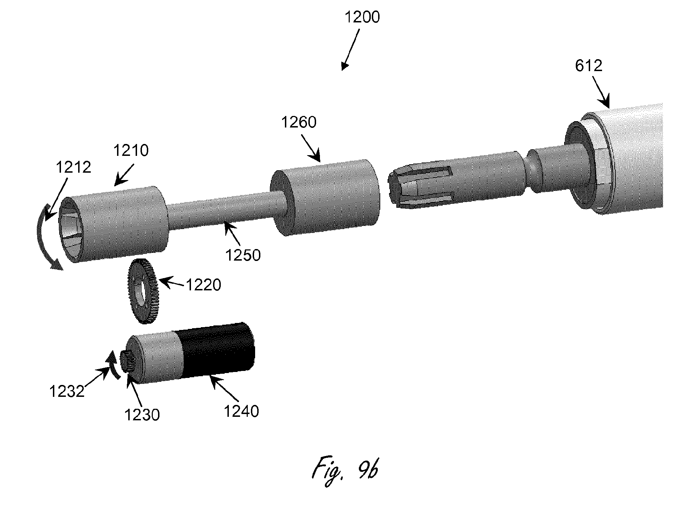

In reference to **Fig.9,** an embodiment of an active controller is shown. In **Fig. 9a,** the controller is shown assembled and in **Fig. 9b,** it is shown disassembled to make more clear the connections between the parts. Similar numbers are used for similar parts in both figures. The controller is connectable via distal connector **1260** to the proximal end of connecting arm **612** (shown disengaged) and, at its proximal end, the controller is connectable via proximal connector **1210** to rods **171** (not shown) of the control unit. Distal connector **1260** is fixed to narrow section **1250,** which fixedly connected to large gear **1220.** Large gear **1220** is fixedly connected to proximal connector **1210.** Large gear **1220** is meshed with small gear **1230,** which is fixedly connected to motor **1240.** The distal end of motor **1240** rests slidably on the proximal end of distal connector **1260,** while the proximal end of motor **1240** rests slidably on the distal end of proximal connector **1210,** fixing motor **1240** in place.

As an example of the functioning of the mechanism, if motor **1240** rotates gear **1230** counterclockwise (CCW) (arrow **1232),** then the motor moves CCW around narrow section **1250** (arrow **1242)** and large gear **1210** rotates clockwise (CW), causing proximal connector **1210** to rotate CW. Since the controller is substantially frictionlessly connected to arms 171, CW rotation of the connectors **1210** and **1260** causes arm **612** to rotate CW, thereby rotating the endoscope unit **200,**

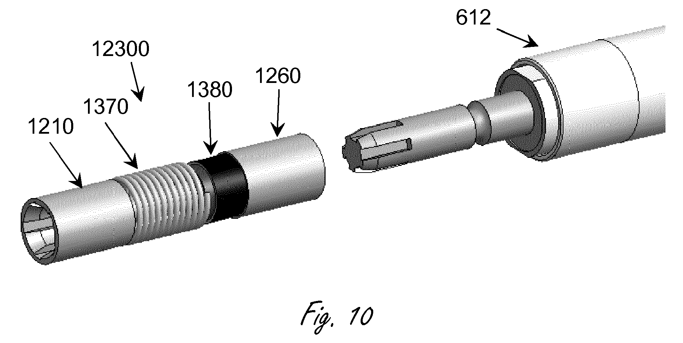

In reference to **Fig. 10,** an embodiment of a passive controller **1200** is shown. In this embodiment, motor **1240** has been replaced by spring **1370** and damping mechanism **1380** and proximal connector **1210** is rotatably connected to narrow section **1250** (not seen)

Spring **1370** is fixedly connected to damping mechanism **1380** and is fixedly connected to proximal connector **1210.** Damping mechanism **1380** is fixedly connected to distal connector **1260,** which is removably connected to arm **612.** Rotation of arm **612** CCW tightens spring **1370,** thereby tending to oppose rotation of arm **612.**

The damping mechanism does at least one of the following: reduces oscillatory motion of the endoscope head; and reduces acceleration of the endoscope head, especially at the beginning and the end of the motion.

In one variant, damping mechanism **1290** comprises a fluid-filled cylinder and a perforated plunger which hugs the inner walls of the fluid-filled cylinder tightly but movably, so that there is little leakage of fluid between the edges of the cylinder and the inner walls of the cylinder. The plunger can move because the fluid can flow through the perforations in it. However, because of the size of the holes and the viscosity of the fluid, the damping mechanism prevents large accelerations of the system and also damps out oscillatory motion in the system.

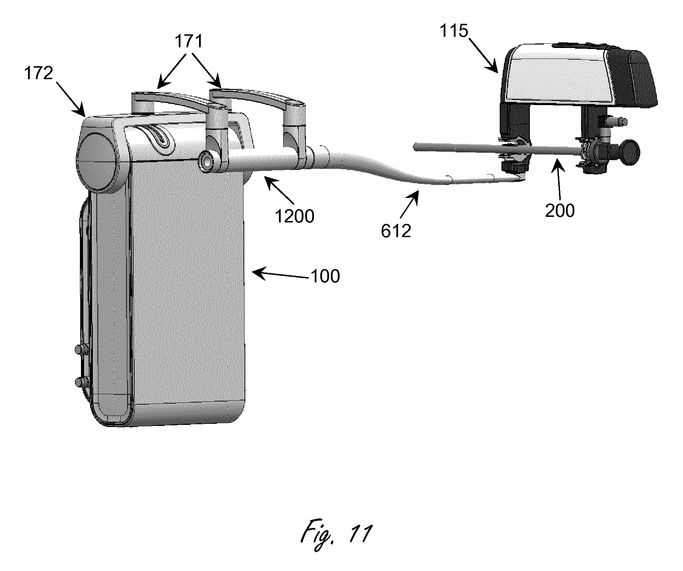

In reference to **Fig. 11,** illustrating another embodiment of the system, first part **100** of the system is connected, as disclosed above, to rods **172** and **171.** Rods **171** are connected to the proximal end of connecting arm **612** via controller **1200,** and the distal end of connecting arm **612** is connected to zoom mechanism **115** and endoscope **200.**

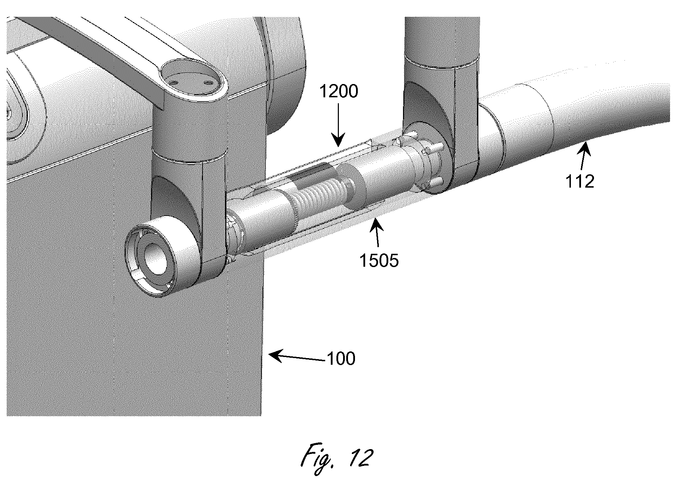

In reference to **Fig. 12,** a combined device **(1200)** is shown, which combines the spring **(1270)** of the passive controller with the motor **(1240)** of the active controller. Also shown **Fig. 12** is cover **1505,** which protects the mechanism and prevents objects, such as the garments of the operating personnel, from interfering with the working of the mechanism. Cover **1505** can also be sterilizable and thereby enable the external portion of the controller to be sterile.

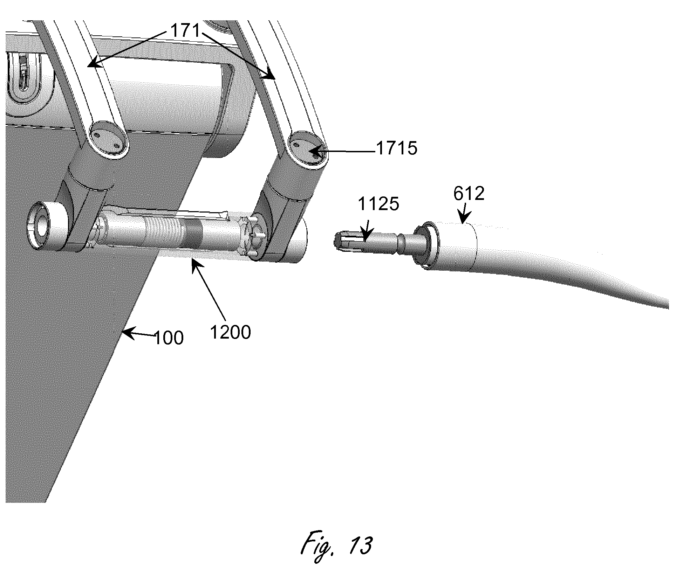

In reference to **Fig. 13,** another view of the controller of **Fig. 13** is shown, showing first part **100** of the system, rods **171,** the proximal end of connecting arm **612,** and controller **1200.** The connector **1125** at the proximal end of connecting arm **612** connects to controller **1200,** which passes through and is supported by rods **171.** Fasteners **1715,** which fix controller **1200** to rods **171,** are also shown.

In some embodiment (e.g., **Fig. 13**) the controller is attached to pivoting support **114;** in other embodiments a controller is attached to or part of pivoting support **113;** in yet other embodiments, a controller is attached to or part of each of the pivoting supports **114** and **113.** In preferred variants of embodiments with two controllers, the controllers are in communication with each other.

In some embodiments, the controller is activated at all times and continually affects movement of at least one of the pivoting supports.

In other embodiments, the controller is activated only if the torque induced by the weight of the endoscope head is large, for example, if the angle θ between the endoscope and the vertical is large, so that the endoscope approaches being parallel to the floor.

In yet other embodiments, the controller is activated if the torque is larger than a predetermined value, so that, for a lightweight enough endoscope, the controller will not be activated at all while, with a large and heavy endoscope, the controller will be activated for most working angles.

In preferred embodiments, the system additionally comprises an automatic assistant in communication with the endoscope which is adapted to maneuver the endoscope.

In preferred embodiments, the system additionally comprises an alignment mechanism adapted to instruct the automatic assistant to align the endoscope.

In another embodiment of the current invention, **FTM 101, STM 102, TTM 103, FOTM 104** and **FTTM 105** are selected in a non-limiting manner from a group consisting, for example, of gearwheels, wheels, crown gears, bevel gears, spur gears, belts, and any combination thereof.

In another embodiment of the current invention, system **100** additionally comprises in a non-limiting manner a quick release handle adapted to disassemble endoscope **200** from system **100** when system **100** is in automatic configuration or in manual configuration

In another embodiment of the current invention, the first mechanism additionally comprises in a non-limiting manner locking means adapted to maintain in a predetermined orientation upon power failure at least one selected from a group consisting, for example, of: **FTM 101, STM 102** and any combination thereof; and to prevent any rotational movement of the same upon power failure.

In another embodiment of the current invention, the second mechanism additionally comprises in a non-limiting manner locking means adapted to maintain in a predetermined orientation upon power failure at least one selected from a group consisting, for example, of: **TTM 103, FOTM 104, FTTM 105** and any combination thereof; and to prevent any rotational movement of the same upon power failure.

**MOS 130** comprises an activation means and a joystick **170** coupled to endoscope **200,** used to manually maneuver endoscope **200** in any direction defined by either one of $\psi$ and $\theta$ as defined above and any combination thereof.

In the manual configuration the physician maneuvers the endoscope (and controls the movement of the same) by means of joystick **170.** According to one embodiment of the present invention, the movement of the joystick is translated into movement of the endoscope.

Upon pressing on the joystick **170** in the directions of arrow **1702** the endoscope moves left or right. The transformation of system form the automatic configuration to the manual configuration, or to the wholly manual means, is obtained by the activation means.

In another embodiment of the current invention, the activation means are selected in a non-limiting manner from a group consisting, for example, of a pressing button, a rotatable knob, a knob, and any combination thereof.

In another embodiment of the current invention, **MOS 130** additionally comprises in a non-limiting manner means for controlling movement of endoscope **200,** adapted to moderate angular velocity in the $\theta$ and $\psi$ directions.

In another embodiment of the current invention, **MOS 130** additionally comprises in a non-limiting manner *n* sensors, where *n* is an integer greater than or equal to one. Sensors are selected in a non-limiting manner from a group consisting, for example, of motion sensors, heat sensors, electric sensors, sound sensors, pressure sensors, optical sensors and any combination thereof. Sensors are adapted to activate in case of power failure or when connected to power.

In another embodiment of the current invention, joystick **170** is characterized in a non-limiting manner by an external surface.

In another embodiment of the current invention, motion sensors detect motion of joystick **170.** Furthermore, motion detection of joystick **170** is used for deactivation of motion of endoscope **200** if the motion's speed is above a predetermined threshold.

In another embodiment of the current invention, motion sensors detect in a non-limiting manner motion upon the external surface of the joystick. Furthermore, motion upon the joystick's external surface is used to operate endoscope **200** in accordance with the motion. Additionally, detection of motion along the joystick is used for deactivation of the motion of endoscope **200** if the speed of the motion along the joystick is above a predetermined threshold.

In another embodiment of the current invention, heat sensors are adapted in a non-limiting manner to sense temperatures in the range of about 35 to about 42 degrees. Said heat sensors are adapted to sense whether a human hand/fingers are activating (i.e., touching) the joystick.

Furthermore, heat sensors enable in a non-limiting manner the activation of **MOS 130** when the heat sensors sense that the temperature is in the range of about 35 to about 42 degrees.

Additionally, heat sensors are adapted in a non-limiting manner to provide a thermal image, where heat sensors are coupled to a processing unit adapted to provide the endoscope user with a thermal image, and the processing unit enables the activation of MOS **130** upon analysis of the image and detection of human hand.

In another embodiment of the current invention, electric sensors are adapted in a non-limiting manner to detect, for example, any of power failure, the electrical conductivity of a human body and any combination thereof. Additionally, human body conductivity sensed by electric sensors enables the activation of the MOS.

In another embodiment of the current invention, sound sensors are adapted in a non-limiting manner to sense predetermined sound patterns. Furthermore, predetermined sound patterns sensed by sound sensors enable the activation of the MOS **130.** Additionally, sound sensors are used to operate endoscope **200** according to predetermined sound patterns (e.g., human voice, predetermined movement commands).

In another embodiment of the current invention, pressure sensors are adapted in a non-limiting manner to sense pressure applied to MOS **130.**

Additionally, when pressure sensed by the pressure sensors is above a predetermined threshold, MOS **130** is either activated or de-activated, and, when the pressure sensed by pressure sensors is below a predetermined threshold, MOS **130** is either activated or de-activated.

In another embodiment of the current invention, optical sensors are adapted in a non-limiting manner to sense visual changes according to predetermined visual patterns. Furthermore, optical sensors enable the activation of MOS **130** according to predetermined visual patterns. Additionally, optical sensors are used to operate endoscope **200** according to predetermined visual patterns.

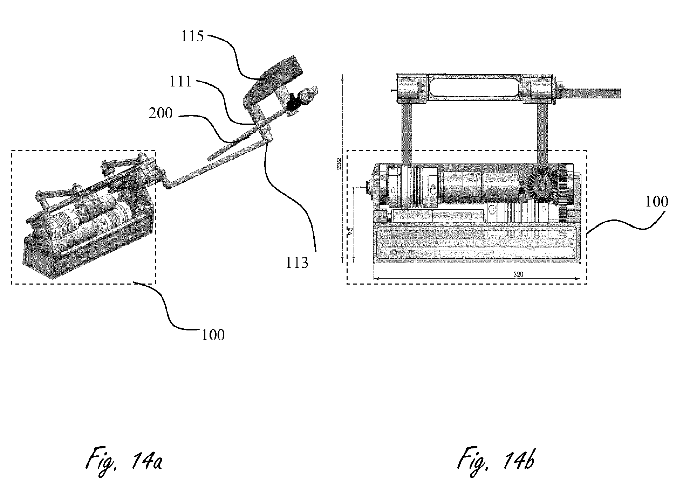

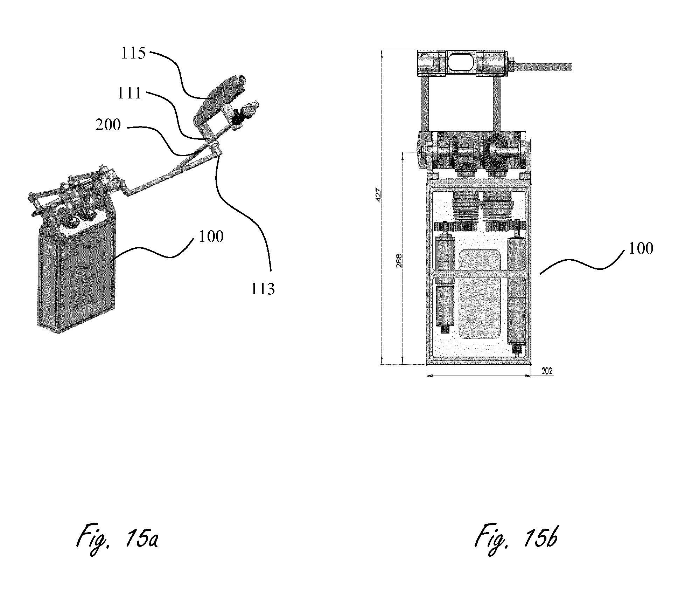

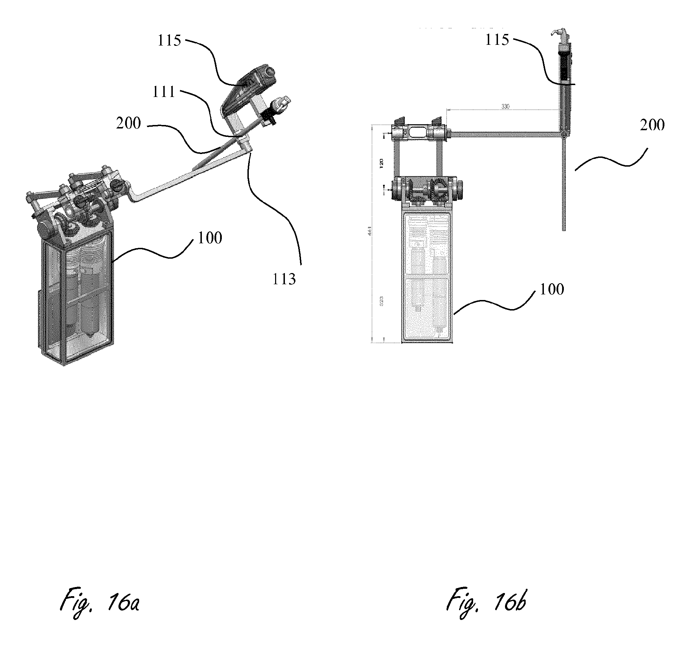

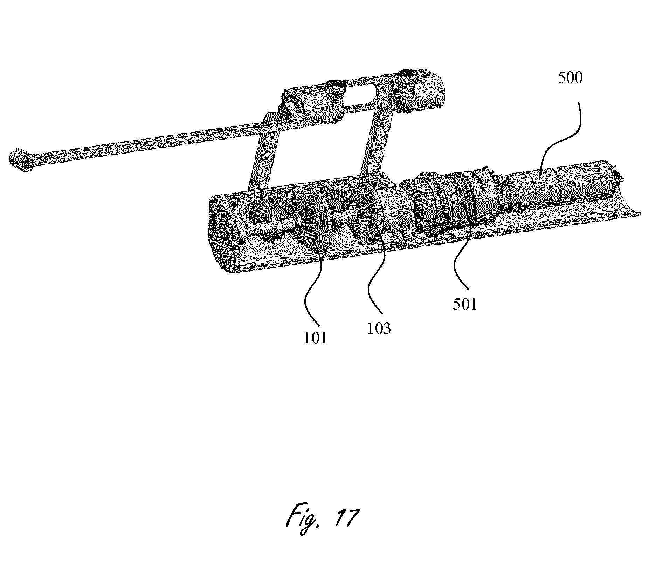

Reference is now made to Figs. **14a** and **14b,** illustrating in a non-limiting manner, from different points of view, the first mechanism and the second mechanism assembled in a horizontal configuration. Reference is now made to Figs. **15a** and **15b,** illustrating in a non-limiting manner different points of view of the first mechanism and the second mechanism assembled in a vertical configuration. Reference is now made to Figs. **16a** and **16b,** illustrating in a non-limiting manner, from different points of view, the first mechanism and the second mechanism assembled in a compact vertical configuration. Reference is now made to Fig. **17** which depicts, in a non-limiting manner, one configuration of the first mechanism and the second mechanism, where first rotation means **106** and second rotation means **107** (shown in Fig. **1)** are unified to a single rotation means **500.**

Said single rotation means **500** is provided with means adapted to switch between rotating first transmission means **101** and third transmission means **103** by a clutch **501.**

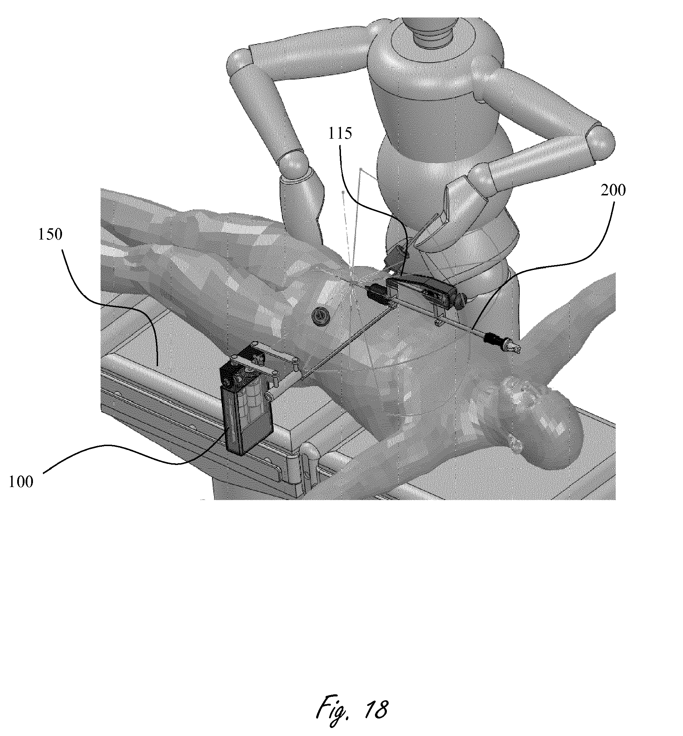

Reference is now made to Fig. **18,** which presents, in a non-limiting manner, attaching means adapted to reversibly couple system **100** to a hospital bed **150.** Attaching means are selected in a non-limiting manner from a group consisting, for example, of mechanical means as defined above, magnetic means as defined above and any combination thereof. Fig. **21** also illustrates a main core concept of the invention, which enables the utilization of the endoscope substantially tangential to the upper surface of the treated organ (e.g. the abdominal cavity

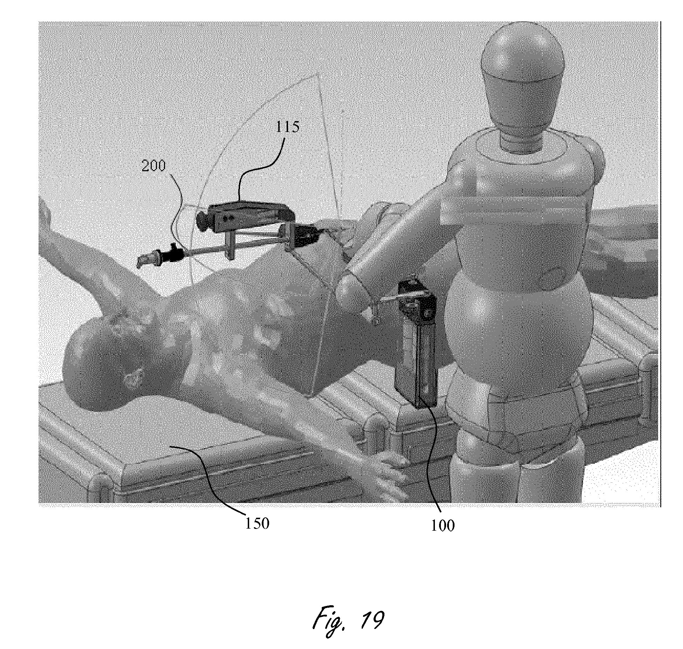

Reference is now made to Fig. **19** which presents, in a non-limiting manner, a possible configuration of system **100,** endoscope **200,** zoom mechanism **115,** and hospital bed **150.** As illustrated in both Figs. **18** and **19,** the system of the present invention enables the operation of the endoscope while the same is substantially parallel to the upper surface of the treated organ (e.g., the abdominal cavity) and, therefore, almost parallel to hospital bed.

According to another embodiment of the present invention a method of maneuvering an endoscope is also provided.

It is an object of the invention to disclose a method for maneuvering an endoscope, the method comprising steps of:

   a. providing a system comprising:

      i. at least one first pivoting support adapted to be pivotally attached to the endoscope; the pivoting support adapted to enable the endoscope to pivot around at least one first axis of rotation;
      ii. at least one second pivoting support in communication with the at least one first pivoting support, the second pivoting support adapted to rotate around at least one axis being substantially orthogonal to the first axis of rotation independently of the first pivoting support; thereby enabling the endoscope to rotate around an insertion point into a body of a subject in at least two orthogonal axes;
      iii. at least one controller attached to either the first pivoting support or the second pivoting support

   b. pivotally attaching the at least one first pivoting support to the endoscope;
   c. positioning the at least one second pivoting support such that at least one axis of rotation of the at least one second pivoting support is substantially orthogonal to at least one axis of rotation of the at least one the first pivoting support;
   d. attaching the at least one controller to at least one of the at least one first pivoting support and the at least one second pivoting support;
   e. maneuvering the endoscope about the penetration point, thereby enabling maneuvering of the endoscope while maintaining alignment of the endoscope about the penetration point.

It is another object of the invention to disclose the method as defined above, comprising a further step of adapting at least one of the pivoting supports to moderate the pivoting of the endoscope around either the first axis of rotation or the second axis of rotation.

It is another object of the invention to disclose the method as defined above, comprising a further step of providing at least one connecting means in connection with the endoscope and at least one of the pivoting supports.

It is another object of the invention to disclose the method as defined above, comprising a further step of adapting at least one of the pivoting supports to constantly apply a counter torque to oppose the torque induced by the endoscope and the connecting means.

It is another object of the invention to disclose the method as defined above, comprising a further step of varying the counter torque as the torque varies, thereby enabling a constant dynamic equilibrium between the endoscope and at least one of the first pivoting support and the second pivoting support.

It is another object of the invention to disclose the method as defined above, comprising a further step of adapting the first pivoting support to enable the endoscope to pivot around one first axis of rotation and adapting the second pivoting support to enable the endoscope to pivot around a second and third axis of rotation, each of the first axis of rotation, the second axis of rotation and the third axis of rotation being substantially perpendicular to the other two axes of rotation.

It is another object of the invention to disclose the method as defined above, comprising a further step of providing the controller with a damping mechanism adapted to prevent oscillation of the system.

It is another object of the invention to disclose the method as defined above, comprising a further step of providing the controller with at least one of an active mechanism and a passive mechanism to provide the dynamic equilibrium.

It is another object of the invention to disclose the method as defined above, comprising a further step of providing the controller with at least one of a group consisting of: a motor and a spring mechanism to provide the dynamic equilibrium.

It is another object of the invention to disclose the method as defined above, comprising a further step of increasing the torque applied by the controller as the angle between the vertical and the endoscope increases.

It is another object of the invention to disclose the method as defined above, comprising a further step of enabling the control to be activated by at least one of a group consisting of: the control is activated at all times, the control is activated when the endoscope's angle is greater than a predetermined value, and the control is activated when the torque on the endoscope is greater than a predetermined value.

It is another object of the invention to disclose the method as defined above, comprising a further step of providing the system with an automatic assistance in communication with the endoscope.

It is another object of the invention to disclose the method as defined above, comprising a further step of adapting the automatic assistance to maneuver the endoscope.

It is another object of the invention to disclose the method as defined above, comprising a further step of providing the system with an alignment mechanism.

It is another object of the invention to disclose the method as defined above, comprising a further step of adapting the alignment mechanism to instruct the automatic assistance to align the endoscope.

It is another object of the invention to disclose the method as defined above, further comprising steps of:

   a. providing a system comprising:

      i. a first mechanism, comprising:

         a) at least one first transmission means **101;** first transmission means **101** defines a first plane; first transmission means **101** is characterized by a first axis of rotation; the first axis of rotation is substantially orthogonal to the first plane;
         b) at least one second transmission means **102;** second transmission means **102** defines a second plane; second transmission means **102** defines a second axis of rotation; the second axis of rotation is substantially orthogonal to the second plane; second transmission means **102** is rotatably connected to first transmission means **101;** where the first plane is substantially orthogonal to second plane; and
         c) at least one first means **106** adapted to rotate first transmission means **101** around the first axis of rotation;

      ii. a second mechanism, comprising:

         a) at least one third transmission means **103;** third transmission means **103** defines a third plane; third transmission means **103** is characterized by a third axis of rotation; the third axis of rotation is substantially orthogonal to the third plane;
         b) at least one fourth transmission means **104;** fourth transmission means **104** defines a fourth plane; fourth transmission means **104** defines a fourth axis of rotation; the fourth axis of rotation is substantially orthogonal to the fourth plane; the fourth transmission means **104** is rotatably connected to third transmission means **103;** the fourth plane is substantially orthogonal to the third plane;
         c) at least one fifth transmission means **105;** fifth transmission means **105** defines a fifth plane; fifth transmission means **105** defines a fifth axis of rotation; the fifth axis of rotation is substantially orthogonal to the fifth plane; fifth transmission means **105** is rotatably connected to fourth transmission means 104; the fifth plane is substantially orthogonal to the fourth plane;
         d) at least one second means **107** adapted to rotate third transmission means **103** around the third axis of rotation;

   b. positioning first transmission means **101** orthogonal to second transmission means **102;** the positioning enables transmission of rotation between first transmission means **101** and second transmission means **102;**

c. positioning third transmission means **103** orthogonal to fourth transmission means **104**; the positioning enables transmission of rotation between third transmission means **103** and fourth transmission means **104**.

d. positioning fourth transmission means **104** orthogonal to fifth transmission means **105**; the positioning enables transmission of rotation between fourth transmission means **104** and fifth transmission means **105**.

e. coupling second transmission means **102** to the endoscope and fifth transmission means **105** to the endoscope; the coupling enables rotation of the endoscope proportional to rotation of second transmission means **102** and fifth transmission means **105**; and,

f. maneuvering the endoscope in at least two degrees of freedom (DOF); maneuvering of the endoscope in the at least two degrees of freedom are in second axis of rotation **141** and in fifth axis of rotation **142**;

wherein maneuvering in a first DOF of the at least two DOF is performed by a step of rotating first transmission means **101** thereby transmitting rotation to the endoscope; wherein maneuvering in a second DOF of at least two DOF is performed by a step of rotating third transmission means **103** thereby transmitting rotation to the endoscope.

It is another object of the invention to disclose the method as defined above, further comprising a step of defining an angle **A** between second axis of rotation **141** and fifth axis of rotation **142**; angle **A** is in the range of about 0 degrees to about 180 degrees.

It is another object of the invention to disclose the method as defined above, further comprising steps of

a. providing at least one zoom mechanism **115**; and

b. maneuvering the endoscope along the main longitudinal axis of the same.

It is another object of the invention to disclose the method as defined above, further comprising a step of selecting at least one of the first and second pivoting supports **(113** and **114)** to be a gimbal.

It is another object of the invention to disclose the method as defined above, further comprising a steps of

a. selecting first transmission means **101,** second transmission means **102,** third transmission means **103,** fourth transmission means **104,** and fifth transmission means **105** from a group consisting of gearwheels, wheels, crown gears, bevel gears, spur gears, belts, and any combinations thereof and

b. providing the system with attaching means adapted to reversibly couple the system to a hospital bed, the attaching means selected from a group consisting of mechanical means, magnetic means and any combination thereof.

It is another object of the invention to disclose the method as defined above, further comprising a step of selecting the mechanical means from a group consisting of a clip, a fastening element, tape, adhesive tape, a snap fastener, a button and any combination thereof.

It is another object of the invention to disclose the method as defined above, further comprising a step of providing, as the magnetic means, a magnetic device comprising at least one magnet and at least one selected from a group consisting of a ferromagnet and a paramagnet; where the magnet is attached to any member of a group consisting of: a hospital bed, the system, and any combination thereof, and at least one selected from a group consisting of a ferromagnet and a paramagnet is attached to at least one member of a group consisting of: a hospital bed, the system, and any combination thereof.

It is another object of the invention to disclose the method as defined above, further comprising a step of defining an angle $\theta$ for rotation in said second plane, the angle $\theta$ to vary between about 0 and about 360 degrees, preferably between about 0 and about 160 degrees, when the system is in its automatic configuration or in its manual configuration.

It is another object of the invention to disclose the method as defined above, further comprising a step of defining an angle $\psi$ for rotation in the fifth plane, the angle $\psi$ to vary between about 0 and about 360 degrees, preferably between about 0 and about 140 degrees, when the system is in its automatic configuration or in its manual configuration.

It is another object of the invention to disclose the method as defined above, further comprising a step of additionally providing the system with a quick release handle adapted to disassemble the endoscope from the system when the system is in its automatic configuration or in its manual configuration.

It is another object of the invention to disclose the method as defined above, further comprising a step of providing the first mechanism with locking means adapted to maintain in a predetermined orientation upon power failure at least one selected from a group consisting of: the first transmission means, the second transmission means and any combination thereof; and to prevent any rotational movement of the same upon power failure.

It is another object of the invention to disclose the method as defined above, further comprising a step of providing the second mechanism additionally with locking means adapted to maintain in a predetermined orientation upon power failure at least one selected from a group consisting of: the third transmission means, the fourth transmission means, the fifth transmission means, and any combination thereof; and to prevent any rotational movement of the same upon power failure.

It is another object of the invention to disclose the method as defined above, further comprising a step of providing at least one manual override system (MOS), adapted upon activation of the same to switch reversibly between a manual configuration, in which the endoscope is moved manually by the operator and an automatic configuration, in which the endoscope is moved automatically by the system and optionally further comprising a step of enabling the same to switch reversibly to a third configuration, in which the endoscope is moved wholly manually by an endoscope assistant.

It is another object of the invention to disclose the method as defined above, further comprising a step of providing at least one joystick, in communication with the endoscope.

It is another object of the invention to disclose the method as defined above, further comprising a step of providing activation means adapted to activate at least one of a group consisting of the system, the joystick and any combination thereof.

It is another object of the invention to disclose the method as defined above, further comprising a step of enabling the joystick to be worn by the joystick user.

It is another object of the invention to disclose the method as defined above, further comprising a step of enabling the activation means to be worn by the MOS user.

It is another object of the invention to disclose the method as defined above, further comprising a step of selecting the activation means from a group consisting of a pressable button, a rotatable knob, a knob, and any combination thereof.

It is another object of the invention to disclose the method as defined above, further comprising a step of enabling the MOS to rotate in the angles $\psi$ and $\theta$.

It is another object of the invention to disclose the method as defined above, further comprising a step of defining angles $\alpha$ and $\beta$ such that the endoscope moves in angular direction $\theta$ when the joystick is moved in direction $\alpha$, and the endoscope moves in angular direction $\psi$ when the joystick is moved in direction $\beta$. It is another object of the invention to disclose the method as defined above, wherein movement of the joystick in a direction selected from a group consisting of $\alpha$, $\beta$ and any combination thereof, is proportional to movement of the endoscope in angular directions $\psi$, $\theta$ and any combination thereof.

It is another object of the invention to disclose the method as defined above, further comprising a step of providing the MOS with means for controlling the endoscope motion, adapted to moderate angular velocity in the $\theta$ and $\psi$ directions.

It is another object of the invention to disclose the method as defined above, further comprising a step of providing the MOS with $n$ sensors, where $n$ is an integer greater than or equal to one.

It is another object of the invention to disclose the method as defined above, further comprising a step of selecting the sensors from of a group consisting of: motion sensors, heat sensors, electric sensors, sound sensors, pressure sensors, optical sensors and any combination thereof.

It is another object of the invention to disclose the method as defined above, further comprising a step selected from at least one of the following: activating the $n$ sensors in case of power failure and activating the $n$ sensors when the system is connected to power.

It is another object of the invention to disclose the method as defined above, further comprising step of detecting the motion of the joystick with motion sensors.

It is another object of the invention to disclose the method as defined above, further comprising a step of using the motion detection of the joystick for deactivation of the motion of the endoscope if the motion's speed is above a predetermined threshold.

It is another object of the invention to disclose the method as defined above, further comprising a step of characterizing the joystick by an external surface.

It is another object of the invention to disclose the method as defined above, further comprising a step of operating the motion sensors to detect motion upon the external surface.

It is another object of the invention to disclose the method as defined above, further comprising a step of operating the endoscope according to the motion upon the external surface.

It is another object of the invention to disclose the method as defined above, further comprising a step of deactivation of the motion of the endoscope when the motion's speed along the joystick is above a predetermined threshold.

It is another object of the invention to disclose the method as defined above, further comprising a step of adapting the heat sensors to sense temperatures in the range of about 35 to about 42 degrees.

It is another object of the invention to disclose the method as defined above, further comprising a step of enabling the activation of the MOS when the heat sensors sense that the temperature is in the range of about 35 to about 42 degrees.

It is another object of the invention to disclose the method as defined above, further comprising a step of adapting the heat sensors to provide at least one thermal image, where the heat sensors are coupled to a processing unit adapted to provide the endoscope user with the thermal image.

It is another object of the invention to disclose the method as defined above, further comprising a step of adapting the electric sensors to sense power failure.

It is another object of the invention to disclose the method as defined above, further comprising a step of adapting the electric sensors to sense electrical conductivity of human body.

It is another object of the invention to disclose the method as defined above, further comprising a step of enabling the activation of the MOS upon sensing the human body's conductivity by electric sensors.

It is another object of the invention to disclose the method as defined above, further comprising a step of adapting the sound sensors to sense predetermined sound patterns.

It is another object of the invention to disclose the method as defined above, further comprising a step of operating the endoscope according to predetermined sound patterns sensed by the sound sensors.

It is another object of the invention to disclose the method as defined above, further comprising a step of adapting the pressure sensors to sense pressure applied to the MOS.

It is another object of the invention to disclose the method as defined above, further comprising a step selected from at least one of a group consisting of: activating the MOS when the pressure sensed by the pressure sensors is above a predetermined threshold and de-activating the MOS when the pressure sensed by the pressure sensors is below a predetermined threshold.

It is another object of the invention to disclose the method as defined above, further comprising a step of adapting the optical sensors to sense visual changes according to predetermined visual patterns.

It is another object of the invention to disclose the method as defined above, further comprising a step of operating said endoscope according to predetermined visual patterns detected by said sensors.

It is another object of the invention to disclose the method as defined above, further comprising a step of enabling the activation of said MOS upon at least one condition selected from a group consisting of: analysis of said thermal image by said processing unit and detection of a human hand, said electric sensors sense said human body conductivity, said sound sensors sense said predetermined sound patterns, and according to said predetermined visual patterns detected by said optical sensors.

It is another object of the invention to disclose the method as defined above, further comprising a step of altering the activation state of said MOS under at least one condition selected from a group consisting of: when said pressure sensed by said pressure sensors is above a predetermined threshold said MOS is activated, and when said pressure sensed by said pressure sensors is below a predetermined threshold, said MOS is de-activated.

It will be apparent to one skilled in the art that there are several embodiments of the invention that differ in details of construction, without affecting the essential nature thereof, and therefore the invention is not limited by that which is illustrated in the figures and described in the specification, but only as indicated in the accompanying claims, with the proper scope determined only by the broadest interpretation of the claims.

**Claims**

1. A system for maneuvering an endoscope, comprising:

   a. at least one first pivoting support adapted to be pivotally attached to said endoscope; said pivoting support adapted to enable said endoscope to pivot around at least one first axis of rotation;
   b. at least one second pivoting support in communication with said at least one first pivoting support, said second pivoting support adapted to rotate around at least one axis being substantially orthogonal to said first axis of rotation independently of said first pivoting support; thereby enabling said endoscope to rotate around an insertion point into a body of a subject in at least two orthogonal axes;
   c. at least one controller attached to either said first pivoting support or said second pivoting support,

   wherein said controller is adapted to provide a constant dynamic equilibrium between said endoscope and at least one of said first pivoting support or said second pivoting support.

2. The system according to claim 1, wherein at least one of said pivoting supports is adapted to moderate said pivoting of said endoscope around either said first axis of rotation or said second axis of rotation.

3. The system according to claim 1, wherein said system further comprises at least one connecting means adapted to connect said endoscope to at least one of said pivoting supports.

4. The system according to claim 3, wherein at least one of said pivoting supports is adapted to constantly apply a counter torque to oppose the torque induced by said endoscope and said connecting means; further wherein said counter torque varies as said torque varies such that there is provided a constant dynamic equilibrium between said endoscope and at least one of said first pivoting support and said second pivoting support.

5. The system according to claim 1, wherein said first pivoting support is adapted to enable said endoscope to pivot around one first axis of rotation and said second pivoting support is adapted to enable said endoscope to pivot around a second and third axis of rotation, each of said first axis of rotation, said second axis of rotation and said third axis of rotation being substantially perpendicular to the other two axes of rotation.

6. The system according to claim 1, wherein said controller further comprises at least one element selected from a group consisting of (a) damping mechanism adapted to prevent oscillation of the system; (b) at least one of an active mechanism and a passive mechanism to provide said dynamic equilibrium; (c) at least one selected from a group consisting of a motor and a spring; and any combination thereof.

7. The system according to claim 1, wherein at least one of the following is being held true (a) said system additionally comprises an automatic assistant in communication with said endoscope adapted to maneuver said endoscope; (b) said system additionally comprises an alignment mechanism; and any combination thereof.

8. The system of claim 1, further comprising:

   a. a first mechanism, comprising:

      i. at least one first transmission means **101;** said first transmission means **101** defines a first plane; said first transmission means **101** is **characterized by** a first axis of rotation; said first axis of rotation is substantially orthogonal to said first plane;
      ii. at least one second transmission means **102;** said second transmission means **102** defines a second plane; said second transmission means is **characterized by** a second axis of rotation **141;** said second axis of rotation **141** is substantially orthogonal to said second plane; said second transmission means **102** is rotatably connected to said first transmission means **101;** where said first plane is substantially orthogonal to second plane; and
      iii. at least one first means **106** adapted to rotate said first transmission means **101** around said first axis of rotation;

   where said first transmission means **101** transmits rotation to said second transmission means **102;** and,
   b. a second mechanism, comprising:

      i. at least one third transmission means **103;** said third transmission means **103** defines a third plane; said third transmission means **103** is **characterized by** a third axis of rotation; said third axis of rotation is substantially orthogonal to said third plane;
      ii. at least one fourth transmission means **104;** said fourth transmission means **104** defines a fourth plane; said forth transmission means defines a fourth axis of rotation; said fourth axis of rotation is substantially orthogonal to said fourth plane; said fourth transmission means **104** is rotatably connected to said third transmission means **103;** where said fourth plane is substantially orthogonal to said third plane;
      iii. at least one fifth transmission means **105;** said fifth transmission means **105** defines a fifth plane; said fifth transmission means defines a fifth axis of rotation **142;** said fifth axis of rotation **142** is substantially orthogonal to said fifth plane; said fifth transmission means **105** is rotatably connected to said fourth transmission means **104;** where said fifth plane is substantially orthogonal to said fourth plane;
      iv. at least one second means **107** adapted to rotate said third transmission means **103** around said third axis of rotation;

   where said third transmission means **103** transmits rotation to said fourth transmission means **104;** where said fourth transmission means **104** transmits rotation to said fifth transmission means **105,**

   wherein said first mechanism and said second mechanism are adapted to rotate said endoscope around at least one said second axis of rotation **141** being substantially orthogonal to said second plane; and around at least one said fifth axis of rotation **142** being substantially orthogonal to said fifth plane, such that said second axis of rotation **141** and said fifth axis of rotation **142** are positioned at an angle A relative to each other; said angle A between said second axis of rotation **141** and said fifth axis of rotation **142** is in the range of about 0 degrees to about 180 degrees.

9. The system according to either one of claims 1 or 8, wherein at least one of the following is being held true (a) said system further comprising at least one zoom mechanism **115,** adapted to maneuver said endoscope along the main longitudinal axis of the same; (b) either one of said first or second pivoting supports **(113** and **114)** is a gimbal; (c) said first transmission means **101,** said second transmission means **102,** said third transmission means **103,** said fourth transmission means **104,** and said fifth transmission means **105** are selected from a group consisting of gearwheels, wheels, crown gears, bevel gears, spur gears, belts, and any combination thereof; and any combination thereof.

10. The system according to claim 1, wherein said system comprises attaching means adapted to reversibly couple said system to a hospital bed, said attaching means selected from a group consisting of mechanical means, magnetic means and any combination thereof; said mechanical means is selected from a group consisting of a clip, a fastening element, tape, adhesive tape, a snap fastener, a button and any combination thereof; said magnetic means comprises a magnetic device, said magnetic device comprising at least one magnet and at least one selected from a group consisting of: a ferromagnet and a paramagnet; where said magnet is attached to at least one member of a group consisting of: a hospital bed, said system, and any combination thereof, and said member of said group consisting of a ferromagnet and a paramagnet is attached to at least one member of a group consisting of: a hospital bed, said system, and any combination thereof.

11. The system according to claim 8, wherein said rotation in said second plane defines an angle $\theta$; said angle $\theta$ varies between about 0 and about 360 degrees, preferably between about 0 and about 160 degrees, when said system is in said automatic configuration or in said manual configuration; further wherein said rotation in said fifth plane defines an angle $\psi$; said angle $\psi$ varies between about 0 and about 360 degrees, preferably between about 0 and about 140 degrees, when system is in said automatic configuration or in said manual configuration.

12. The system according to claim 1, wherein said system additionally comprises a quick release handle adapted to disassemble said endoscope from said system when said system is in said automatic configuration or in said manual configuration.

13. The system according to claim 8, wherein at least one of the following is being held true (a) said first mechanism additionally comprises locking means adapted to maintain in a predetermined orientation upon power failure at least one selected from a group consisting of: said first transmission means, said second transmission means and any combination thereof; and to prevent any rotational movement of the same upon power failure; (b) said second mechanism additionally comprises locking means adapted to maintain in a predetermined orientation upon power failure at least one selected from a group consisting of: said third transmission means, said fourth transmission means, said fifth transmission means, and any combination thereof; and to prevent any rotational movement of the same upon power failure; and any combination thereof.

14. The system according to claim 1, additionally comprising at least one manual override system (MOS), adapted upon activation of the same to switch reversibly between a manual configuration, in which the endoscope is moved manually by the operator and an automatic configuration, in which the endoscope is moved automatically by the system, and optionally adapted to switch reversibly to a wholly manual configuration, in which the endoscope is moved wholly manually by an endoscope assistant.

15. The system according to claim 14, wherein at least one of the following is being held true (a) said system additionally comprising at least one joystick, in communication with said endoscope; (b) said system additionally comprising activation means adapted to activate at least one of a group consisting of said system, said joystick and any combination thereof; (c) said activation means is adapted to be worn by said MOS operator; (d) said activation means is selected from a group consisting of a pressable button, a rotatable knob, a knob, and any combination thereof.

16. The system according to claim 14, wherein said MOS additionally comprises $n$ sensors, where $n$ is an integer greater than or equal to one; wherein said sensors are selected from of a group consisting of: motion sensors, heat sensors, electric sensors, sound sensors, pressure sensors, optical sensors and any combination thereof.

17. The system according to claim 16, wherein at least one of the following is being held true (a) said motion sensors detect motion of said joystick; wherein said motion detection of said joystick is used to deactivate said motion of said endoscope if said motion's speed is above a predetermined threshold; (b) said motion sensors detect motion upon the external surface of said joystick; wherein said motion upon the external surface of said joystick is used to operate said endoscope according to said motion upon said external surface; (c) said heat sensors are adapted to sense temperature in the range of about 35 to about 42 degrees; (d) said heat sensors enable the activation of said MOS when said heat sensors sense said temperature is in the range of about 35 to about 42 degrees; (e) said heat sensors are adapted to provide a thermal image, where said heat sensors are coupled to a processing unit adapted to provide said endoscope user with said thermal image; (f) said electric sensors are adapted to sense at least one of a group consisting of: power failure, connection to power, and electrical conductivity of a human body; (g) said sound sensors are adapted to sense predetermined sound patterns; wherein said sound sensors are used to operate said endoscope according to said predetermined sound patterns; (h) said optical sensors are adapted to sense visual changes according to predetermined visual patterns; wherein said optical sensors are used to operate said endoscope according to said predetermined visual patterns; (i) said pressure sensors are adapted to sense pressure applied to said MOS.

18. The system according to claims 16 and 17 wherein said MOS is activated upon at least one condition selected from a group consisting of: analysis of said thermal image by said processing unit and detection of human hand, said electric sensors sense said human body conductivity, said sound sensors sense said predetermined sound patterns, and according to said predetermined visual patterns detected by said optical sensors.

Fig. 1

Fig. 2a

111
113
Free Joints
112
114
115
200
180

Fig. 2b

Fig. 3a

Fig. 3b

Fig. 3c

Fig. 4a

Fig. 4b

200

180

*Fig. 5a*

200

180

*Fig. 5b*

651

200

653

612

114

100

111

*Fig. 6*

*Fig. 7*

*Fig. 8*

*Fig. 9a*

Fig. 9b

Fig. 10

Fig. 11

*Fig. 12*

*Fig. 13*

Fig. 14a

Fig. 14b

Fig. 15a

Fig. 15b

115

111

200

100

113

*Fig. 16a*

115

330

120

466.1

323

200

100

*Fig. 16b*

500

501

101 103

Fig. 17

*Fig. 18*

*Fig. 19*

Europäisches
Patentamt

European
Patent Office

Office européen
des brevets

# EUROPEAN SEARCH REPORT

Application Number

EP 14 15 1130

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2009/312600 A1 (SHOLEV MORDEHAI [IL]) 17 December 2009 (2009-12-17) * paragraphs [0012] - [0025], [0032] - [0034], [0095], [0096], [0100]; figures 2,5-9 * ----- | 1-18 | INV. A61B19/00 |
| Y | WO 2010/122563 A1 (M S T MEDICAL SURGERY TECHNOLO [IL]; SHOLEV MORDEHAI [IL]) 28 October 2010 (2010-10-28) * page 26, lines 14-19,; figures 15,16,24 * * page 27, line 18 - page 28, line 5 * * page 29, lines 16,26 * ----- | 1-9, 11-18 | |
| Y | EP 2 246 006 A2 (AKTORMED GMBH [DE]) 3 November 2010 (2010-11-03) * paragraphs [0002], [0011] - [0013]; figure 2 * ----- | 10 | |
| A | EP 2 347 785 A1 (NAT UNIV CORP NAGOYA INST TECH [JP]; UNIV NAGOYA NAT UNIV CORP [JP]; N) 27 July 2011 (2011-07-27) * paragraph [0065] * ----- | 1-18 | TECHNICAL FIELDS SEARCHED (IPC) A61B |

The present search report has been drawn up for all claims

| Place of search | Date of completion of the search | Examiner |
|---|---|---|
| The Hague | 24 March 2014 | Lee, Ronan |

EPO FORM 1503 03.82 (P04C01)

## ANNEX TO THE EUROPEAN SEARCH REPORT
## ON EUROPEAN PATENT APPLICATION NO.

EP 14 15 1130

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2014

| Patent document cited in search report | | Publication date | Patent family member(s) | | Publication date |
|---|---|---|---|---|---|
| US 2009312600 | A1 | 17-12-2009 | EP | 2073685 A2 | 01-07-2009 |
| | | | US | 2009312600 A1 | 17-12-2009 |
| | | | WO | 2008035345 A2 | 27-03-2008 |
| WO 2010122563 | A1 | 28-10-2010 | EP | 2421424 A1 | 29-02-2012 |
| | | | US | 2012041263 A1 | 16-02-2012 |
| | | | WO | 2010122563 A1 | 28-10-2010 |
| EP 2246006 | A2 | 03-11-2010 | DE | 102009018917 A1 | 18-11-2010 |
| | | | EP | 2246006 A2 | 03-11-2010 |
| EP 2347785 | A1 | 27-07-2011 | EP | 2347785 A1 | 27-07-2011 |
| | | | JP | 5403785 B2 | 29-01-2014 |
| | | | JP | 2010094235 A | 30-04-2010 |
| | | | US | 2011264038 A1 | 27-10-2011 |
| | | | WO | 2010044377 A1 | 22-04-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006100501 A

- JP 06063003 B